# EUROPEAN PATENT SPECIFICATION

(11) **EP 1 256 624 B1**
(45) Date of publication and mention of the grant of the patent: **17.10.2007**
(21) Application number: 01901562.7
(22) Date of filing: 30.01.2001
(51) Int. Cl.: C12M 1/34, C12Q 1/06, G01N 1/30, G01N 33/58

(54) **KIT FOR DETECTING MICROORGANISMS, APPARATUS FOR QUANTIFYING MICROORGANISMS AND METHOD FOR QUANTIFYING MICROORGANISMS**
REAGENZIENSATZ ZUR DETEKTION VON MIKROORGANISMEN, VORRICHTUNG ZUR QUANTIFIZIERUNGVON MIKROORGANISMEN UND VERFAHREN ZUR QUANTIFIZIERUNG VON MIKROORGANISMEN
TROUSSE DE DETECTION DE MICRO-ORGANISMES, APPAREIL ET PROCEDE DE QUANTIFICATION DE MICRO-ORGANISMES

(30) Priority: 31.01.2000 JP 2000021656; 31.01.2000 JP 2000021657; 29.03.2000 JP 2000090372; 11.12.2000 JP 2000375643
(43) Date of publication of application: 13.11.2002
(60) Divisional application: 06007141.2
(73) Proprietor: Matsushita Ecology Systems Co., Ltd., Kasugai-shi Aichi 486-8522 (JP)
(72) Inventor: Shimakita, Tomonori, Komaki-shi Aichi 486-8522 (JP); Tashiro, Yoshikazu, Kasugai-shi Aichi 486-0833 (JP); Nashimoto, Kazuo, Kasugai-shi Aichi 486-0833 (JP)
(74) Representative: Körfer, Thomas
(86) International application number: PCT/JP2001/000594
(87) International publication number: WO 2001/057180

(56) References cited:
- EP-A- 0 545 284
- EP-A- 0 713 087
- EP-A- 0 816 513
- EP-A2- 0 436 897
- WO-A-98/22618
- WO-A-99/58948
- FR-A- 2 636 646
- JP-A- 4 232 459
- JP-A- 5 184 579
- JP-A- 7 155 166
- JP-A- 11 094 747
- JP-A- 11 113 562
- JP-A- 11 146 798
- JP-A- 11 178 596
- JP-B1- 47 016 787
- US-A- 4 900 934
- US-A- 5 314 805
- MCFETERS GORDON A ET AL: "Physiological assessment of bacteria using fluorochromes" JOURNAL OF MICROBIOLOGICAL METHODS, vol. 21, no. 1, 1995, pages 1-13, XP002309063 ISSN: 0167-7012
- DARZYNKIEWICZ ET AL.: EXPERIMENTAL CELL RESEARCH, vol. 249, 1999, pages 1-12,

## Description

### TECHNICAL FIELD

The present invention relates to a microorganism counting process capable of counting the color-developed microorganisms.

### BACKGROUND ART

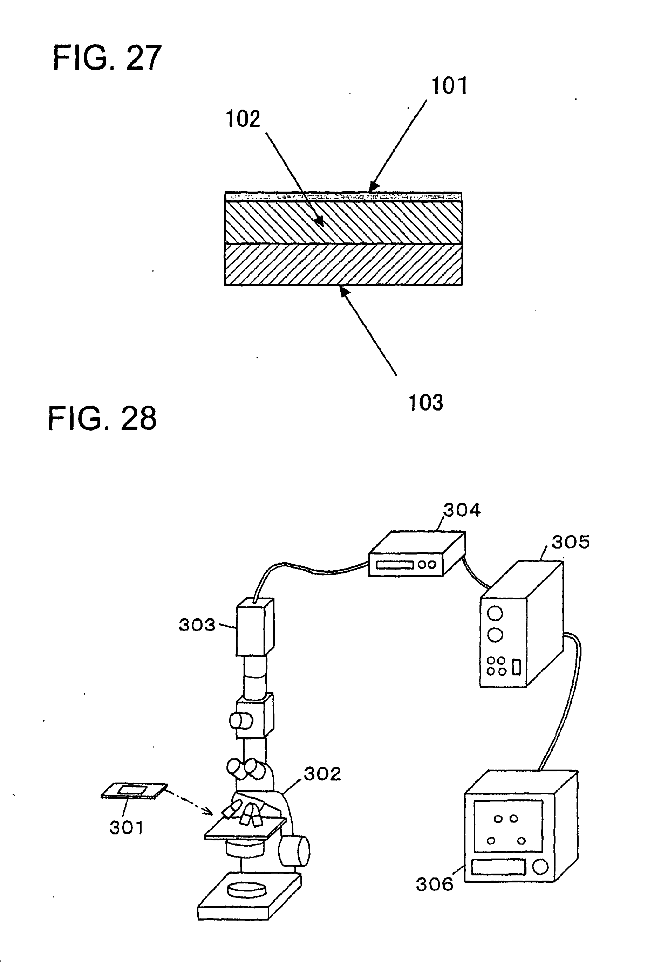

As for a kit for detecting the microorganisms, there are known a self-adhesive sheet as described in Japanese Patent Application Laid-open No.10-70975, and a plate counting process, which is a common process for determining the number of deposited cells. The detail of such detecting kit is shown in Fig.27. The self-adhesive sheet 101 is comprised of a self-adhesive layer 102 and a support 103. Microorganisms in a specimen are brought into a contact with the self-adhesive layer 102 and collected on a surface of the self-adhesive layer 102. An antibody and/or an aqueous solution of a color-developing substrate for microorganisms to be detected is brought into contact with the surface of the self-adhesive layer 102, and the color development of the microorganisms on the surface of the self-adhesive layer 102 is observed visually or by a microscope, thereby confirming the presence or absence of the microorganisms and the number of the microorganisms.

There is also known a deposited-bacterium measuring kit (not shown) based on a plate counting process as one of culturing processes, which are common processes for determining the number of bacteria or fungi. The deposited-bacterium measuring kit is adapted to judge the presence or absence of microorganisms and count the number of microorganisms by bringing the surface of a culture medium into direct contact with a specimen, collecting microorganisms on the surface of the culture medium, culturing the microorganism deposited to the surface of the culture medium as they are, and observing them visually or by a microscope or the like.

Such conventional microorganism detecting kit requires steps of bringing the self-adhesive sheet into contact with the specimen, collecting the microorganisms and then applying a reagent. For this reason, a time and a technique are required for an examining operation. An immune process for detecting particular microorganisms using an antibody has a high sensitiveness, because the antibody is bonded specifically with an antigen. In the immune process, however, the use of the antibody is essentially required and for this reason, there are problems in respect of the nature itself of the antibody such as the production and management of the antibody and an immune reaction detecting means. The immune process is not capable of judging the life or death of cells. In addition, if the dyeing of the antibody is used, the self-adhesive layer is dyed to a dyed color and after the dyeing, colors other than the specimen deposited to the self-adhesive layer are required to be removed. Further, the antibody and the color-developing substrate are used, and the entire area to be detected is dyed and for this reason, particular microorganisms can be detected, but cannot be detected simultaneously with the common bacteria.

There are conventionally known apparatus for counting microorganisms, which is designed to apply exciting light directly to a sample containing microorganisms, acquire fluorescent rays emitted from the fluorescent substances produced in the microorganisms as image data, measure a concentration of the fluorescent substances in the microorganisms and measure the activity of the microorganisms from the addition of the intensity of the fluorescent ray, as described in Japanese Patent Application Laid-open No. 5-111394. The detail of such microorganism counting process is shown in Fig.28. A suspension of methanogens germs is placed on a preparation 301, and exciting light 302 is applied to the suspension. Fluorescent rays generated are recognized using ahigh-sensitiveness television camera 303 and VTR 304 and stored. The intensity of the intended fluorescent ray is detected from an image picture projected on a monitor television 306 using a picture-processing device 305.

Such conventional microorganism counting process cannot be suited for microorganisms producing no fluorescent substance. In addition, in the conventional microorganism counting process, microorganisms incapable of producing a fluorescent substance because of a weaker activity are not detected in some oases, even if they are target microorganisms. The activity or number of the microorganisms cannot be necessarily detected. The microorganism counting apparatus is used in food factories in many oases. In such case, it is an important factor for the management how many are all microorganisms present rather than particular microorganisms. Therefore, for as the standard of living microorganisms on a fresh vegetable, the number of common bacteria is 1,000,000/ml or less. According to the Introduction of a latest HACCP, not only the examination of the number of living germs on a food itself but also the examination in a factory itself and in an operating procedure, e.g., the examination of living germs on a wall, a floor surface, cooking devices such as a chopping board and a kitchen knife have been conducted, and the management of a food itself and the management of environments are important. However, the conventional technique is complicated and requires a specialized knowledge and thus, it is not true that anybody can measure microorganism easily in a field using the conventional technique. Even in respect of the apparatus, exciting light is applied to fluorescent substances produced by microorganisms, and fluorescent rays emitted from the fluorescent substances are analyzed by processing of a picture. For this reason, a technique for enlarging the picture at high magnifications as in a microscope is required.

The common culturing process for detecting the deposited germs suffers from a problem that a lot of time is taken for culturing deposited microorganisms. Further, some of types of microorganisms may be not bred on a culture medium having the microorganisms deposited thereon in some oases, which may cause an underestimation. Furthermore, the culturing process detects only living germs and for this reason, the number and the life or death of microorganisms existing in the environments cannot be necessarily confirmed by the culturing process.

There are such conventionally known microorganism counting processes and apparatus, in which an added compound is permeated into microorganism bodies and then decomposed by an enzyme reaction in the microorganism bodies, and microorganisms emitting fluorescent rays are detected, as described in Japanese Patent Application Laid-open No. 10-323197. These microorganisms are recognized as living microorganisms. In addition, the numbers of living and dead microorganisms are grasped by using a reagent for dyeing the dead cells in combination with an above-described reagent. There are other conventionally known techniques, in which particular microorganisms are detected in a luminescent manner using an antibody, as described in Japanese Patent Application Laid-open No.7-140148, and in which the number of particular microorganisms and the numbers of living and dead microorganisms are grasped using a reagent for dyeing the microorganisms non-specifically in combination with an antibody.

In a food sanitation field or the like, it is desired to detect particular microorganisms promptly. Coliform baciteria and particular E.coli are measured as the particular microorganisms, and depending on the type of food, particular microorganisms such as staphylococcus are measured. However, in a situation in which particular microorganisms coexist together with common bacteria or in a situation in which impurities exist, it is required that the particular microorganisms are separated or separated and cultured using a separating means, or the impurities are removed. The common bacteria and the particular microorganisms cannot be counted simultaneously.

In such conventional microorganism counting process and apparatus, a process for discriminating microorganisms promptly described in Japanese Patent Application Laid-open No. 10-323197 uses a fluorescent reagent comprising fluorescein or the derivative thereof and a fluorescent dye comprising propidium iodide under limited conditions. However, the fluorescent reagent comprising fluorescein or the derivative thereof is subject to a course in which it is decomposed by esterase produced by microorganisms to emit rays, and hence, the fluorescent reagent largely depends on the activity of the enzyme. Namely, an enzyme activation temperature or the substrate specificity varied depending on the type of microorganisms is exemplified as an error influence factor. Therefore, in environments in which the same type of microorganisms only exists, a high-sensitiveness measurement is expected, but there is a high possibility that an underestimation or an overestimation may be caused in actual service environments. The esterase may be produced even outside microorganism cells. In such a case, the microorganisms are integrated with a background and for this reason, there is a possibility that an accurate detection cannot be achieved. Therefore, there is a problem that even if the fluorescent reagent is used in combination with the propidium iodide, or the double dyeing is carried out, the numbers of living and dead microorganisms cannot be necessarily grasped.

On the other hand, the microorganism counting process described in Japanese Patent Application Laid-open No. 7-140148 employs an antibody to detect specific microorganisms and a dyeing reagent to be non-specifically bonded to microorganisms. In this process, it is requisite to use the antibody, but the antibody is high in sensitiveness, because it is bonded specifically, and on the other hand, the life and the death cannot be discriminated. Further, a process using this technique cannot discriminate the life and death of microorganisms dyed by the non-specifically dyeing reagent contained in a specimen.

It is also desired to measure particular microorganisms and common bacteria simultaneously and promptly. EP 0 713 087 A1 discloses a microbiological counting process involving staining microbes on a solid support with vital dyes, scanning the microbes with exciting light from a laser, the laser spot being 4-14 microns in size, and detecting intensity of the resultant emitted light from each spot. The detection of total viable bacteria in water is described using a fluorescent marker, fluorescein succimidyl ester, in an aliquot of water. Fluorescein succimidyl ester is a substrate for esterase that all microorganisms have.

It is an object of the present invention to provide a microorganism counting process, wherein an error-influencing factor due to the enzyme activation of microorganisms can be eliminated, whereby the number of microorganisms can be counted precisely.

Further, it is an object of the present invention to provide a microorganism counting process, wherein the integration of microorganisms with a measuring background can be prevented, whereby the common bacteria and particular microorganisms can be measured simultaneously.

Yet further, it is an object of the present invention to provide a microorganism counting process, wherein the wavelength of exciting light and fluorescent ray having a particular wavelength and providing the staining by the exciting light can be controlled by carrying out the staining by fluorescent rays, whereby cells to be detected can be counted precisely.

Yet further, it is an object of the present invention to provide a microorganism counting process, which is capable of counting the number of only living microorganisms by using a compound developing a color by the reaction with a substance derived from microorganisms as a compound for staining living cells.

Yet further, it is an object of the present invention to provide a microorganism counting process, which is capable of judging the presence or absence of living microorganisms at a higher sensitiveness by using a compound developing a color by the reaction with an enzyme protein as a substance derived from microorganisms.

Yet further, it is an object of the present invention to provide a microorganism counting process, which is capable of judging the presence or absence of particular microorganisms at a higher sensitiveness by using a compound developing a color by the reaction with an enzyme protein as a substance derived from particular microorganisms.

Yet further, it is an object of the present invention to provide a microorganism counting process, which is capable of counting the number of particular microorganisms by bringing a compound for staining microorganisms by the reaction with a substance derived from the particular microorganisms into contact with a specimen and detecting the wavelength and the amount of color developed.

### DISCLOSURE OF THE INVENTION

To achieve the above objects, the present invention provides a microorganism counting process comprising the steps of bringing 4-methylumbelliferyl-β-D-galactoside and/or 4-methylumbelliferyl-β-D-glucuronide for coloring Coliform bacteria into contact with a specimen, and detecting each one of the Coliform bacteria by detecting the fluorescent light emitted by the excitation by ultraviolet rays, wherein said detection is performed by applying the exciting light which is emitted from a light source section and collected in a region of a very small given area of 0.2 to 7.0 µm per side as determined based on the size of Coliform bacteria and counting the number of Coliform bacteria based on the wavelength of the fluorescent coloured rays derived from 4-methylumbelliferron which is a decomposition product of 4-methylumbelliferyl-β-D-galactoside and/or 4-methylumbelliferyl-β-D-glucuronide in each Coliform bacteria in a light-amount measuring section converting the rays into electric signals.

The microorganism counting process has a feature that the staining or coloration is fluorescent. Thus, according to the present invention, only the microorganisms stained by fluorescent rays can be counted at a high sensitiveness.

The present invention provides a microorganism counting process comprising the step of bringing a4-methylumbelliferyl-β-D-galactoside and/or 4-methylumbelliferyl-β-D-glucuronide for staining Coliform bacteria by the reaction with a substance derived from Coliform bacteria into contact with a specimen to detect the Coliform bacteria from a wavelength and an amount of color developed. Thus, according to the present invention, it is possible to reliably recognize the Coliform bacteria by specifically bonding the compound to the Coliform bacteria and further to precisely grasp the number of Coliform bacteria from an amount of fluorescent color different from the color of the reagent.

### BRIEF DESCRIPTION OF THE DRAWINGS

Fig. 1 is an illustration of the arrangement of a microorganism detecting kit according to Example 1 of the present invention.
Fig.2 is an illustration of the arrangement of a detecting kit according to Example 2 of the present invention, which includes a reagent-containing vessel and a specimen contact vessel.
Fig.3 is an illustration of the arrangement of a detecting kit including reagent-containing sheets.
Fig.4 is an illustration of the arrangement of a detecting kit with carriers retained on each of reagent-containing sheets according to Example 3 of the present invention.
Fig.5 is an illustration of the arrangement of a detecting kit with reagent-supporting carriers retained in a separated manner on each of reagent-containing sheets according to Example 4 of the present invention.
Fig.6 is an illustration of the arrangement of a detecting kit including a light-separating film according to Example 5 of the present invention.
Fig.7 is an illustration of the arrangement of a liquid specimen detecting kit according to Example 6 of the present invention.
Fig.8 is a curve of attenuation of fluorescent light according to Example 7 of the present invention.
Fig. 9 is a diagram indicating threshold values with respect to the intensity of fluorescent light according to Example 8 of the present invention.
Fig.10 is an illustration of the arrangement of a bacterium-recognizing means according to Example 9 of the present invention.
Fig.11 is a diagram of a locus A of exciting light on a specimen according to Example 10 of the present invention.
Fig. 12 is a diagram of a locus B of exciting light on the specimen according to Example 10 of the present invention.
Fig.13 is a diagram showing a waveform model according to Example 10 of the present invention.
Fig.14 is a diagram showing a waveform model according to Example 10 of the present invention, when there are microorganisms existing adjacent one another.
Fig.15 is a fluorescent ray extinction model according to Example 10 of the present invention.
Fig.16 is a diagram showing a microorganism counting apparatus according to Example 10 of the present invention.
Fig. 17 is a diagram showing a binary model according to Example 10 of the present invention.
Fig.18 is a diagram showing a microorganism counting apparatus according to Example 11 of the present invention.
Fig.19 is a diagram showing a specimen contact means according to Example 12 of the present invention.
Fig.20 is a diagram for explaining a process for counting living and dead cells and dead cells according to Example 13 of the present invention.
Fig.21 is a block diagram of a microorganism counting apparatus according to Example 14 of the present invention.
Fig.22 is a graph showing a calibration curve for living and dead cells and electric signal output in Examples 13 and 14.
Fig.23 is a graph showing a calibration curve for dead cells and electric signal output in Examples 13 and 14.
Fig. 24 is a diagram for explaining a process for counting living and dead cells, dead cells, living cells and Coliform bacteria according to Example 15 of the present invention.
Fig.25 is a graph showing a calibration curve for living cells having a high activity and electric signal output according to Examples 15 and 16 of the present invention.
Fig.26 is a graph showing a calibration curve for Coliform bacteria and electric signal output according to Examples 15 and 16 of the present invention.
Fig.27 is an illustration of the arrangement of a prior art detecting kit.
Fig.28 is a view of the arrangement of a prior art microorganism measuring apparatus.

### BEST MODE FOR CARRYING OUT THE INVENTION

A microorganism counting process including the process of the invention comprises the steps of bringing one or more of a first compound for staining living and dead cells, a second compound for staining dead cells at a wavelength different from the above-described staining, and a third compound for staining living cells at a wavelength different from the above-described staining; and 4-methylumbelliferyl-β-D-galactoside and/or 4-methylumbelliferyl-β-D-glucuronide for staining Coliform bacteria at a wavelength different from the above-described staining by the reaction with a substance derived from Coliform bacteria, into contact with a specimen, and detecting both or one of the living cells and the dead cells and the Coliform bacteria simultaneously from a difference between the wavelengths and amounts of colors developed. The living and dead cells, the dead cells, the living cells and the Coliform bacteria can be detected simultaneously, whereby the counting of polluting microorganisms in medical and food sanitation fields and the monitoring of microorganisms in environments can be carried out promptly. For example, the number of common bacteria can be counted in food fields by the counting of the living and dead calls, the counting of the dead cells and the counting of the living cells and further, the states of foods after being sterilized can be grasped by measuring the dead cells. In addition, because Coliform bacteria discharge a specific enzyme protein called β-galaotosidase, the presence of Coliform bacteria is ascertained by using 4-methylumbelliferyl-β-D-galactoside, which reacts with the enzyme protein, and hence, the microorganism pollution can be examined in medical and food sanitation fields.

The staining or coloration is fluorescent, thereby providing an effect that the interference of the wavelength of colored rays can be prevented, and the colored rays can be visually recognized simply. Further, the precise number of cells can be counted.

The first compound is one capable of being bonded to nucleic acid. Thus, living and dead cells can be counted to a one-cell level by bonding of the first compound to nucleic acid, and hence, the living and dead cells can be counted precisely at a high sensitiveness even in the presence of impurities such as metabolites from microorganisms, dusts and inorganic substances.

The second compound is one capable of being bonded to nucleic acid. Thus, dead cells can be counted to a one-cell level by bonding of the second compound to nucleic acid, and hence, the dead cells can be counted precisely at a high sensitiveness even in the presence of impurities such as metabolites from microorganisms, dusts and inorganic substances.

The third compound is one for staining microorganisms by the reaction with a substance derived from microorganisms in the specimen, thereby providing an effect that the presence or absence of living microorganisms can be confirmed by the reaction of the third compound with a substance derived from microorganisms such as metabolites from microorganisms. In addition, the substance derived from microorganisms is an enzyme protein and thus, the presence or absence of living microorganisms can be judged at a higher sensitiveness.

The substance derived from Coliform bacteria is an enzyme protein. In general, the enzyme protein is a substance resulting from the metabolism of living cells and hence, the living Coliform bacteria can be counted without destruction of living cell membranes in a pretreatment.

In the microorganism counting process of the invention, a compound for staining Coliform bacteria by the reaction with a substance derived from Coliform bacteria is brought into contact with a specimen, and Coliform bacteria are detected from a wavelength and an amount of color developed. Thus, a detecting reagent can be supplied in a suitable amount to the specimen, and the Coliform bacteria can be counted at a high safety and a high sensitiveness without direct or indirect touching of an operator with the specimen.

### EXAMPLES

A microorganism detecting kit and a microorganism counting apparatus with which the process of the invention can be carried out and a microorganism counting process according to the present invention will now be described in detail by way of embodiments, but the present invention is not limited in any way to the description below.

Example 1 of the present invention will be described below.

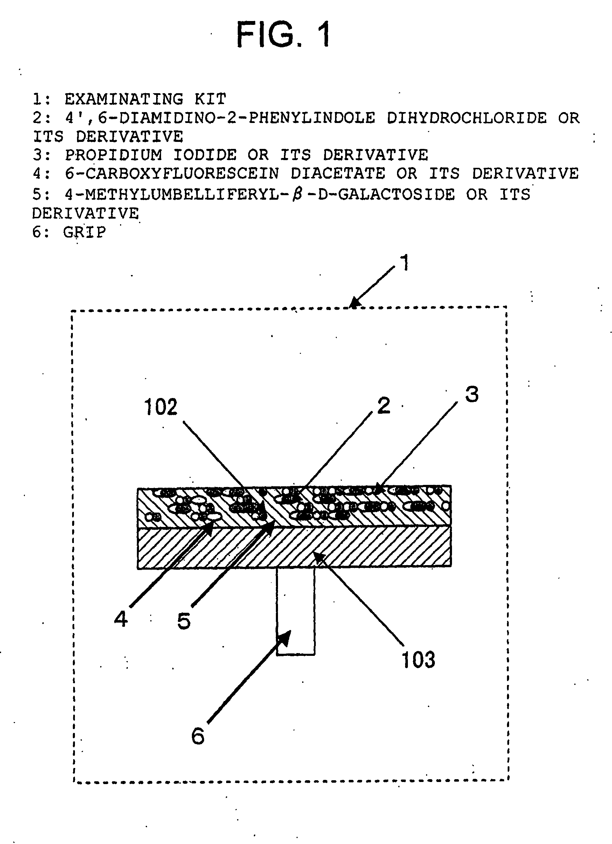

A microorganism detecting kit is shown in Fig.1. Referring to Fig. 1, the detecting kit 1 is comprised of a self-adhesive layer 102 having the following compounds incorporated therein: 4',6-diamidino-2-phenylindole dihydrochloride (the derivative thereof may be used) 2 as a first compound for staining or coloring living and dead cells, propidium iodide (the derivative thereof may be used) 3 as a second compound for staining dead cells at a wavelength different from the above-described staining, 6-carboxyfluorescein diacetate (the derivative thereof may be used) 4 as a third compound for staining living cells at a wavelength different from the above-described staining, and 4-methylumbelliferyl-β-D-galactoside (the derivative thereof may be used) 5 as a fourth compound for staining Coliform bacteria at a wavelength different from the above-described staining by reacting with a substance derived from particular microorganisms, a support 103, and a grip portion 6. The self-adhesive layer 102 includes an agar culture medium containing a meat essence, peptone and sodium chloride and agar, and is formed by mixing the compounds 2, 3, 4 and 5 together and solidifying the resulting mixture after being sterilized. In Fig. 1, all of the compounds 2, 3, 4 and 5 are incorporated, but only one of the compounds may be incorporated, or two of the compounds may be incorporated, or three of the compounds may be incorporated.

In a process for examining cells or microorganisms, in order to deposit cells or microorganisms deposited to the specimen to the self-adhesive layer 102 including the agar culture medium containing the compounds, a rugged surface of a specimen and the self-adhesive layer 102 are pressed using the grip portion 6, whereby the specimen and the self-adhesive layer 102 including the agar culture medium are brought into close contact with each other by the fixed support 103.

After the cell or microorganisms deposited to the specimen are deposited to the self-adhesive layer 102 including the agar culture medium, the compounds 2, 3, 4 and 5 contained in the agar culture medium react with the cell or microorganisms to become individually bonded to the cell or microorganisms to stain or color them. Living and dead cells, dead cells and living cells, which exist, can be confirmed by counting the colored cells.

The 4',6-diamidino-2-phenylindole dihydrochloride for staining or coloring the living and dead cells and the propidium iodide for staining or coloring the dead cells are passed through cell membranes and bonded to nucleic acid.

The 4' ,6-diamidino-2-phenylindole dihydrochloride, after being bonded, absorbs exciting light at an exalting wavelength of 359 nm to emit light having a fluorescent wavelength of 461 nm to color the living and dead cells. The propidium iodide absorbs exciting light at an exciting wavelength of 535 nm to emit light having a fluorescent wavelength of 617 nm to color only the dead cells.

Further, the 6-carboxyfluorescein diacetate is decomposed by esterase produced by cells to produce a fluorescein, thereby emitting fluorescent rays as a result of application of exciting light.

The 4-methylumbelliferyl-β-D-galaotoside reacts with β-galactosidase produced by Coliform bacteria to produce 4-methylumbelliferone, thereby emitting fluorescent rays as a result of application of exciting light.

In an actual use example, sterilized normal agar culture medium containing 4',6-diamidino-2-phenylindole dihydrochloride, propidium iodide, 6-carboxyfluorescein diacetate and 4-methylumbelliferyl-β-D-galactoside, which are detecting reagents, was to be formed a self-adhesive layer, and the self-adhesive layer was brought into direct contact with a wall surface of a drain tank in an air conditioner by using the grip and placed onto a microscope adapted to emit exciting light. Then, exciting light was applied to the surface of the self-adhesive layer. Cells irradiated by the exciting light are colored or stained at individual wavelengths, and the number of individual cells emitting fluorescent rays was counted visually through a light-separating film for separating the fluorescent rays. Germs were collected from the specimen and measured in 2 or 3 minutes. For comparison, microorganisms on the wall surface of the drain tank are collected and cultured in a standard agar food stamp (made by Nissui, Co.) for detecting common bacteria and in a deoxycholate food stamp (made by Nissui, Co.) for detecting Coliform bacteria, and the number of detected cells was counted. However, the food stamps could not detect living and dead cells and dead cells and could not culture thermophiles and special bacteria and for this reason, the number of cells detected was smaller than that detected by the microorganism detecting kit according to the present invention. The number of living Coliform bacteria detected was equal to that of living Coliform bacteria detected by using the microorganism detecting kit,

As described above, amounts of rays emitted from the living and dead cells and the dead cells are counted from differences between wavelengths of colored fluorescent rays and between amounts of rays emitted, thereby counting the numbers of the living cells and the dead cells. Further, the presence or absence of Coliform bacteria and living cells can be confirmed.

To count the deposited germs, they can be detected at 200 to 1,000 magnifications by a magnifying lens such as a microscope.

The mixture after being sterilized was cooled and solidified to form the agar culture medium, but a liquid culture medium with agar removed can be used to confirm the presence or absence of living cells.

The Coliform bacteria were used as the particular microorganisms.

The agar culture medium was used as a specimen contact means, but a water-soluble polymer such as gum arabic and gelatin, polyvinyl alcohol made by polymerizing a water-soluble monomer such as vinyl alcohol and acrylic acid, polyacrylic acid, and a polymer comprising two or more molecules of a water-soluble monomer can be used, if cells can be deposited thereto from a specimen.

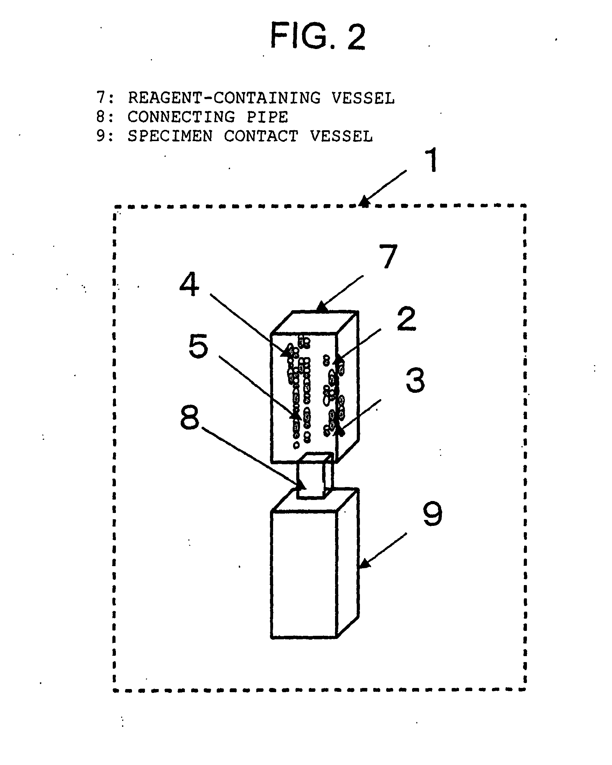

Example 2 of the present invention will be described below. An arrangement according to Example 2 is shown in Fig. 2. This detecting kit 1 includes a reagent-containing vessel 7, into which the following compounds are incorporated: 4',6-diamidino-2-phenylindole dihydrochloride 2 as a first compound for staining or coloring living and dead cells, propidium iodide 3 as a second compound for staining dead cells at a wavelength different from the above-described staining, 6-carboxyfluorescein diacetate 4 as a third compound for staining dead cells at a wavelength different from the above-desoribed staining, and 4-methylumbelliferyl-β-D-galactoside 5 capable of measuring Coliform bacteria as a fourth compound for staining particular microorganisms at a wavelength different from the above-described staining by reacting with a substance derived from particular microorganisms. The reagent-containing vessel 7 is constructed in such a manner it is connected to a specimen contact vessel 9 by a connecting pipe 8. Although not shown, an aqueous solution such as physiological saline or the like may be filled in the specimen contact vessel 9 in some cases. In Fig. 2, all of the compounds 2, 3, 4 and 5 were incorporated, but only one of the compounds may be incorporated, or two of the compounds may be incorporated, or three of the compounds may be incorporated.

In the detecting kit 1, an aqueous solution containing cells or microorganisms or microorganisms collected from a specimen by an applicator or the like are poured into the specimen contact vessel 9, or dissolved into an aqueous solution within the specimen contact vessel 9. The 4' ,6-diamidino-2-phenylindole dihydrochloride 2, propidium iodide 3, 6-carboxyfluorescein diacetate 4 and 4-methylumbelliferyl-β-D-galactoside 5 capable of measuring Coliform bacteria 5 contained in the reagent-oontaining vessel 7 are mixed with cells contained in the specimen contact vessel 9 as a specimen contact means through the connecting pipe 8, and the numbers of dead cells, living and dead cells, living cells and particular microorganisms are counted from colors developed by the reagents.

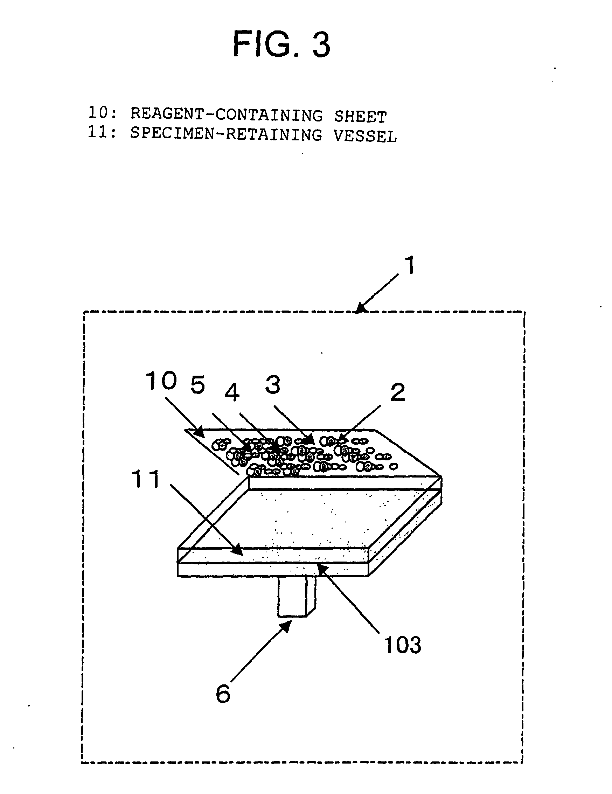

Even if a detecting kit is formed by using a reagent -containing sheet 10 in place of the reagent-containing vessel and using a specimen-retaining vessel 11 in place of the specimen contact vessel, as shown in Fig.3, a similar effect is provided.

In an actual use example, a detecting kit was used, which was formed using a normal agar culture medium as a specimen contact means, and using a detecting-reagent containing section formed by a film retaining a solution containing 4',6-diamidino-2-phenylindole dihydrochloride, propidium iodide, 6-carboxyfluorescein diacetate and 4-methylumbelliferyl-β-D-galactoside. The normal agar culture medium as the specimen contact means was brought into contact with a wall surface of a drain tank in an air conditioner and after the contact, the film containing the detecting reagents was brought into close contact with the surface of normal agar culture medium in contact with the wall surface. The specimen contact means and the detecting-reagent containing section left in contact with each other were placed onto a microscope capable of emitting exciting light, and exciting light was applied to them. Cells irradiated by the exciting light were stained or colored at individual wavelengths, and the number of individual cells emitting fluorescent rays was counted visually through a light-separating film for separating fluorescent rays. Germs were collected from the specimen and measured in 2 or 3 minutes. For comparison, microorganisms on the wall surface of the drain tank are collected and cultured in a standard agar food stamp (made by Nissui, Co.) for detecting common bacteria and in a deoxycholate food stamp (made by Nissui, Co.) for detecting Coliform bacteria, and the number of detected cells was counted. However, the food stamps could not detect living and dead cells and dead cells and could not culture thermophiles and special bacteria and for this reason, the number of cells detected was smaller than that detected by the microorganism detecting kit. The number of living Coliform bacteria detected was equal to that of living Coliform bacteria detected by using the microorganism detecting kit.

With the above-described arrangement, the movement of the compound to the specimen due to the direct contact of the compound to the specimen can be prevented, and microorganisms existing in the specimen can be detected.

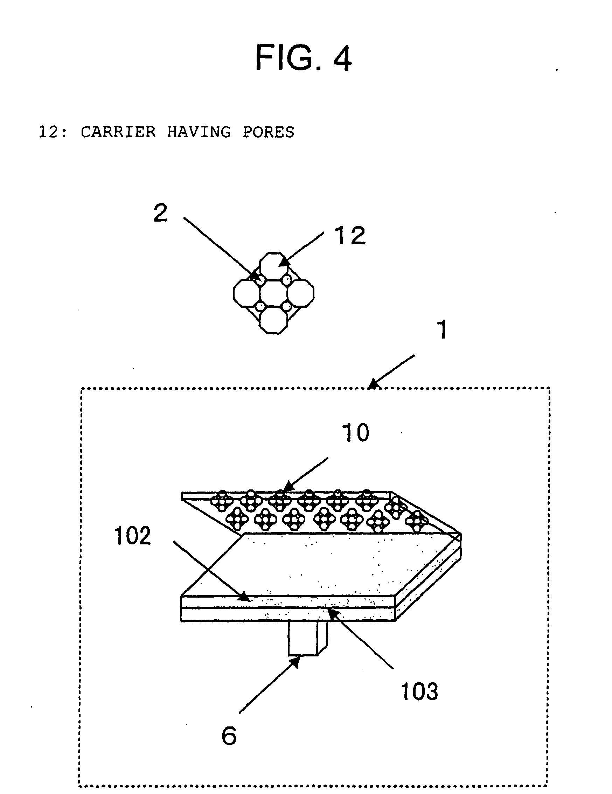

Example 3 of the present invention will be described below. An arrangement is shown in Fig. 4. A detecting kit 1 in Example 3 is comprised of a reagent-oontaining sheet 10 retaining, on its surface, carriers 12 made by using activated carbon as a starting material and each providing with pores and each having 4',6-diamidino-2-phenylindole dihydrochloride 2 supported thereon, a self-adhesive layer 102 which is a specimen contact means, a support 103 retaining the self-adhesive layer 102, and a grip 6 for bringing a specimen into close contact with the self-adhesive layer 102.

With the above-described arrangement, the 4',6-diamidino-2-phenylindole dihydrochloride 2 supported in the pores in the carriers 12 is protected from the oxidation due to a warm heat provided by the natural leaving, ultraviolet rays contained in natural light, or air, and the detecting kit 1 can be preserved for a long time.

It should be noted that by supporting, on a carrier having pores such as the carriers 12 having the pores, propidium iodide as the second compound for staining the dead cells at the wavelength different from the above-described staining, 6-carboxyfluorescein diacetate as the third compound for staining the living cells at the wavelength different from the above-described staining, and 4-methylumbelliferyl-β-D-galactoside capable of measuring Coliform bacteria, as the fourth compound for stating the particular microorganisms at the wavelength different from the above-described staining by the reaction with the substance derived from particular microorganisms, the preservation of them can be facilitated, and the detecting sensitiveness can be enhanced by an increase in area of contact with the specimen.

In the example, the 4',6-diamidino-2-phenylindole dihydrochloride was supported on the carriers 12 made by using activated carbon as the starting material and having the pores, but even if propidium iodide, 6-oarboxyfluoresoein diacetate and 4-methylumbelliferyl-β-D-galactoside capable of measuring Coliform bacteria may be supported in the form of a mixture on activated carbon 10, a similar effect can be provided.

The carrier 12 is a carrier having pores, but if the carrier is one having pores such as zeolite and silica gel, a similar effect can be provided.

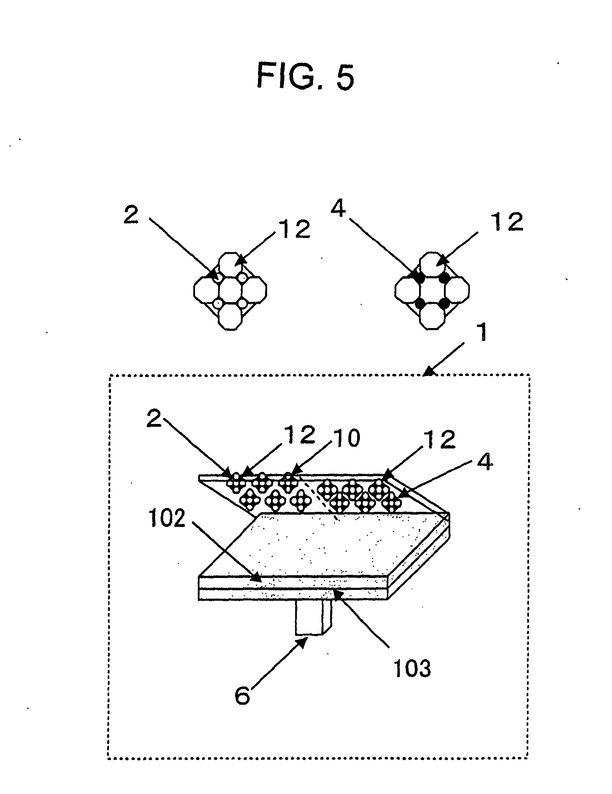

Example 4 of the present invention will be described below. An arrangement is shown in Fig.5.

A detecting kit 1 in Example 4 is comprised of a reagent-retaining layer 10 retaining, in a separated manner on its surface, carriers 12 made by using activated carbon as a starting material and each providing with pores and each having 4',6-diamidino-2-phenylindole dihydrochloride 2 supported thereon as a first compound for staining living and dead cells, and carriers 12 made by using activated carbon as a starting material and each providing with pores and each having 6-carboxyfluorescein diacetate 4 supported thereon as a second compound for stating living cells, a self-adhesive layer 102, a support 103 retaining the self-adhesive layer 102, and a grip 6 for bringing a specimen into close contact with the self-adhesive layer 102.

With the above arrangement, the fluorescent reagents are moved from the carriers 12 contained on the reagent-retaining layer 10 to living and dead cells or dead cells, or to Coliform bacteria or living cells, which is deposited to the self-adhesive layer 102 from the specimen, to dye or stain the various cells. In this case, the reagent-retaining layer 10 is bisected, and the carriers 12 retaining the reagents in the above combination are retained in the resulting separated sections of the reagent-retaining layer 10. To carry out the detection, light having a wavelength peculiar to each of the fluorescent reagents is applied to each of the separated sections to stain intended cells.

Thus, it is possible to detect the presence or absence of Coliform bacteria, the number of all germs, the number of living germs and the number of dead germs at one time and to grasp the state of the specimens in a short time.

It should be noted that the carrier is not limited to the activated carbon.

In addition, if even one type of the reagent is supported on the carrier, a useful effect can be provided.

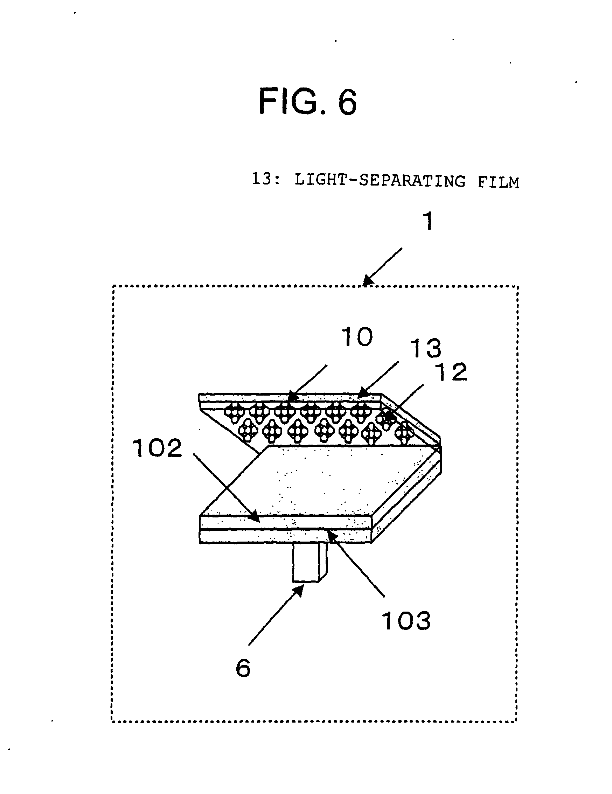

Example 5 of the present invention will be described below. An arrangement is shown in Fig.6.

A detecting kit 1 in Example 5 is comprised of a self-adhesive layer 102 adapted to be brought into direct contact with a specimen to collect cells deposited to the specimen, a support 103 for supporting the self-adhesive layer 102, a grip 6 for enhancing the operability of the detecting kit, carriers 12 containing a reagent colored by the presence of cells in pores, a reagent-containing sheet 10 for retaining the carriers 12, and a light-separating film 13 for separating applied exciting light into light having an intended wavelength.

With the above arrangement, living and dead cells, dead cells and particular microorganisms deposited to the self-adhesive layer 102 are stained or dyed by the staining reagents supported in the carriers 12, and exciting light is applied through the light-separating film 13 to the stained cells and microorganisms. The exciting light stains the cells deposited to the self-adhesive layer 102 through the light-separating film 13 and hence, even if the exciting light is a light of a wider wavelength emitted from a light source, the cells can be detected and counted only by the detecting kit.

The light-separating film 13 is not only available to separate the exciting light but also available to separate fluorescent rays.

The light-separating film 13 may be comprised a film for separating the exciting light and a film for separating the fluorescent rays, which are superposed onto each other. In this case, the separation of the exciting light and the separation of the fluorescent rays produced by the exciting light can be carried out at one time.

The light-separating film 13 may be mounted on the support 103.

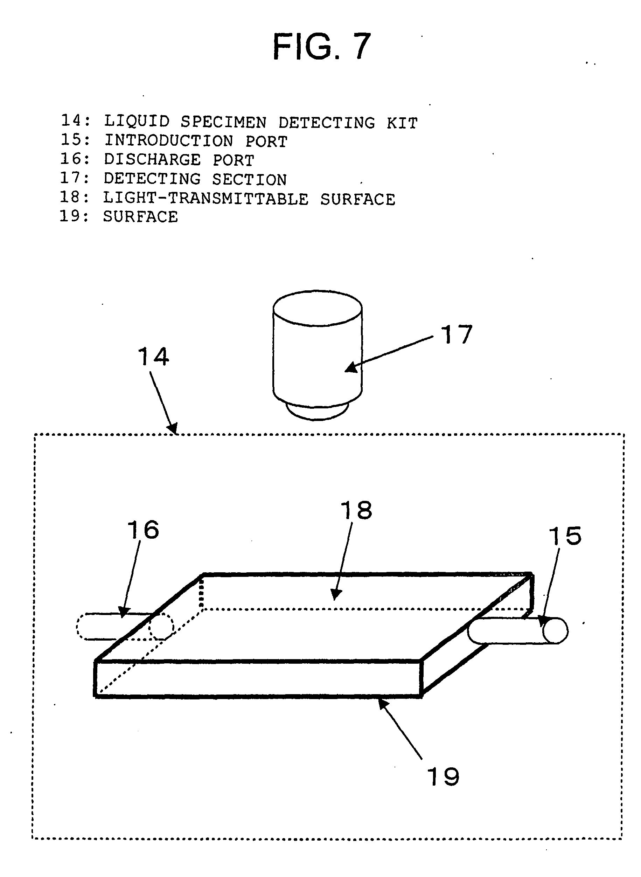

Example 6 of the present invention will be described below. An arrangement is shown in Fig.7.

A liquid specimen detecting kit 14 in Example 6 includes an introduction port 15 for throwing a liquid specimen, and a discharge port 16 for discharging a surplus amount of liquid specimen and air previously contained in the liquid specimen detecting kit 14. The liquid specimen detecting kit 14 is of a structure in which a surface, on which at least a detecting section 17 exists, is a light-transmittable surface 18, so that microorganisms can be detected in the detecting section. Further, a surface 19 opposed to the light-transmittable surface 18 has a structure in which it is in a parallel-positional relationship to the light-transmittable surface 18 and it is not inclined.

With the above arrangement, even if the specimen is liquid, it can be retained in a given space. In addition, when microorganisms contained in the liquid specimen have been introduced into the liquid specimen detecting kit 14, the microorganisms can be diffused in a given region without being superposed one on another, because the distance between the light-transmittable surface 18 and the surface 19 located in an opposed relation to the surface 18 are maintained at a given thickness. Thus, it is possible to prevent the underestimation due to the superposition and to count the microorganisms precisely by a detecting means placed on the detecting section 17.

It should be noted that the sensitiveness for detecting microorganisms can be enhanced by mounting a filter in front of the introduction port 15 in order to remove foreign matters.

In addition, the light-transmittable surface 18 can be formed so that it does not influence the exciting light such as ultraviolet rays, thereby preventing the obstruction of the exciting light.

The distance between the light-transmittable surface 18 and the surface 19 located in an opposed relation to the surface 18 is in a range of 0.5 to 15 µm. Thus, it is possible to prevent the superposition of microorganisms and to achieve the precise discrimination.

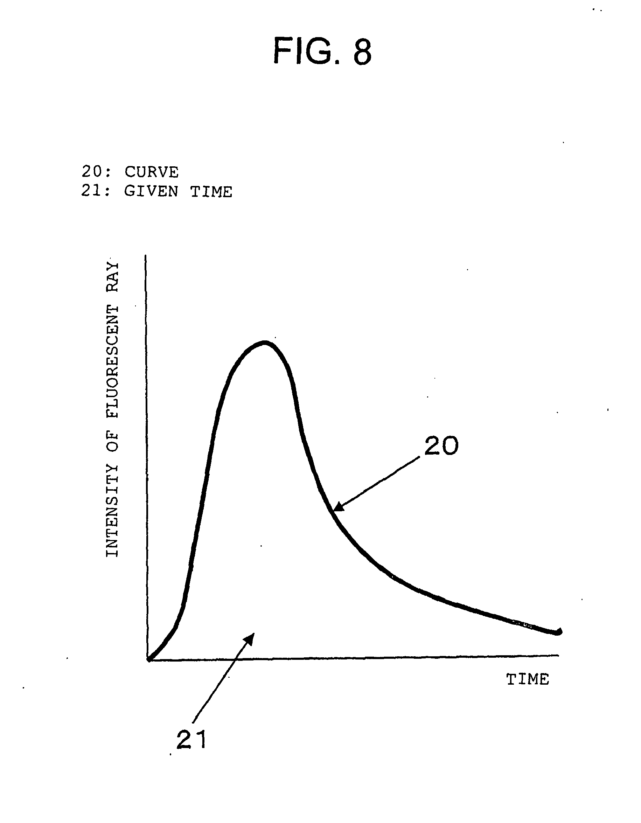

Example 7 of the present invention will be described below. Fig.8 is a curve of attenuation of fluorescent light.

When exciting light is continuously applied to a fluorescent coloring matter, the intensity of fluorescent light emitted by the exciting light forms a curve of attenuation as shown in the curve 20 due to the fading. A given time 21 for maintaining the intensity including and around the maximum intensity of fluorescent light existing in the curve of attenuation is provided in Fig.8.

With the above arrangement, for the intensity of fluorescent light attenuated as shown in the curve 20, even when counting after attenuation and not being counted, the given time 21 for maintaining the intensity including and around the maximum intensity of fluorescent light is provided, and the addition and counting are carried out within such time, whereby the mis-recognition due to the fading of the fluorescent coloring matter can be prevented to enable the precise counting.

The given time 21 can be set to be peculiar to the coloring matter, and may be changed depending on the type of the coloring matter.

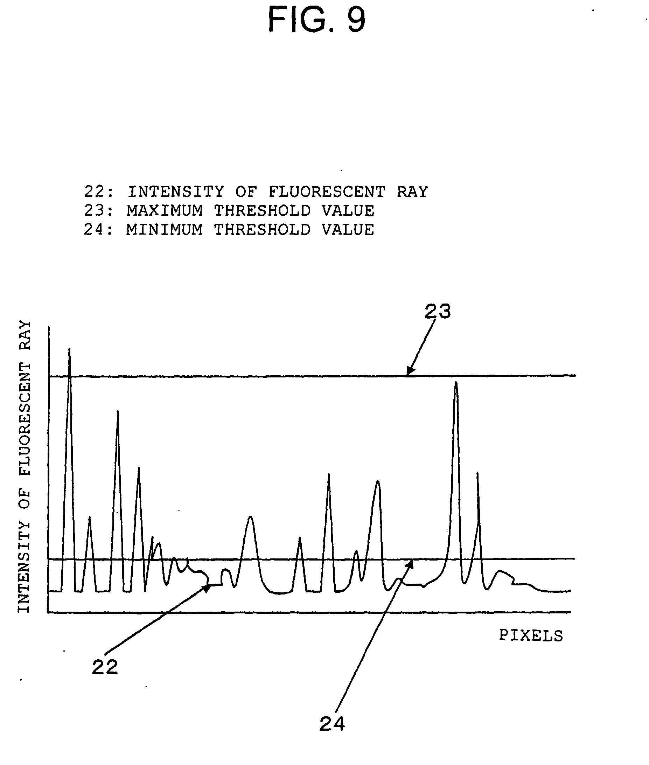

Example 8 of the present invention will be described below. Fig.9 is a diagram indicating threshold values with respect to the intensity of fluorescent light.

When microorganisms existing on a specimen contact means and stained or colored to emit rays have been detected, an intensity 22 of fluorescent rays as shown in Fig. 9 can be obtained. A maximum threshold value 23 and a minimum threshold value 24 derived from the intensity of fluorescent rays of microorganisms are provided for the intensity 22, and values of intensity 22 of fluorescent rays existing between the maximum and minimum threshold values 23 and 24 are recognized as fluorescent rays derived from microorganisms and added all together to count the microorganisms.

With the above arrangement, peaks having intensity values of fluorescent rays existing between the maximum and minimum threshold values 23 and 24 derived from the intensity of fluorescent rays of the microorganisms are recognized as microorganisms, and other values are determined as provided by foreign matters and are not counted, thereby enabling the precise counting.

The threshold values may be varied depending on the coloring matter, and a preset time suitable to the varied values may be provided.

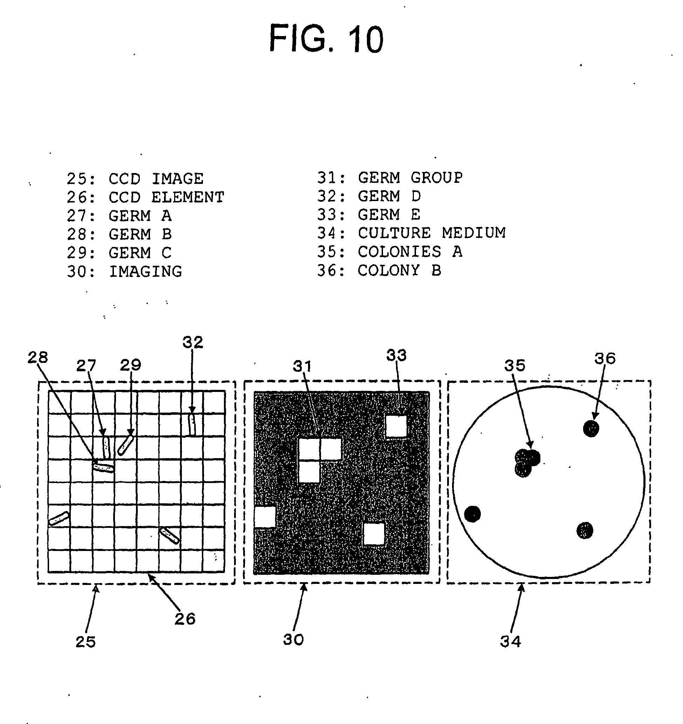

**Example 9** of the present invention will be described below. An arrangement is shown in Fig.10.

Microorganisms existing on a specimen contact means and colored to emit light are shown in a CCD image 25. When they are recognized by a CCD element 26, rays emitted from microorganisms are recognized within pixels. In this case, when a pixel with a germ B28 recognized therein and a pixel with a germ C29 recognized therein exist around a pixel with a germ A27 recognized, the germ A27, the germ B28 and the germ C29 are recognized as a germ group 31, as shown in an imaging diagram 30. When a germ such as a germ D32 is recognized within one pixel, it is recognized as one germ B33, as shown in the imaging diagram 30, and the germs in the pixels are counted in an adding manner. When the germs existing at locations on the CCD image 25 have been cultured, colonies A35 and a colony B36 as shown in a culture medium 34 are formed.

With the above arrangement, when a large number of germs exist, adjacent germs are recognized as one germ group 31 and counted as one bright point in an adding manner. Thus, when the counting of the germs are carried out in the culture process which is one of the prior art processes is carried out, the correlation to the prior art process can be provided in consideration of the fact that the germ group is recognized as one colony.

Example 10 of the present invention will be described below.

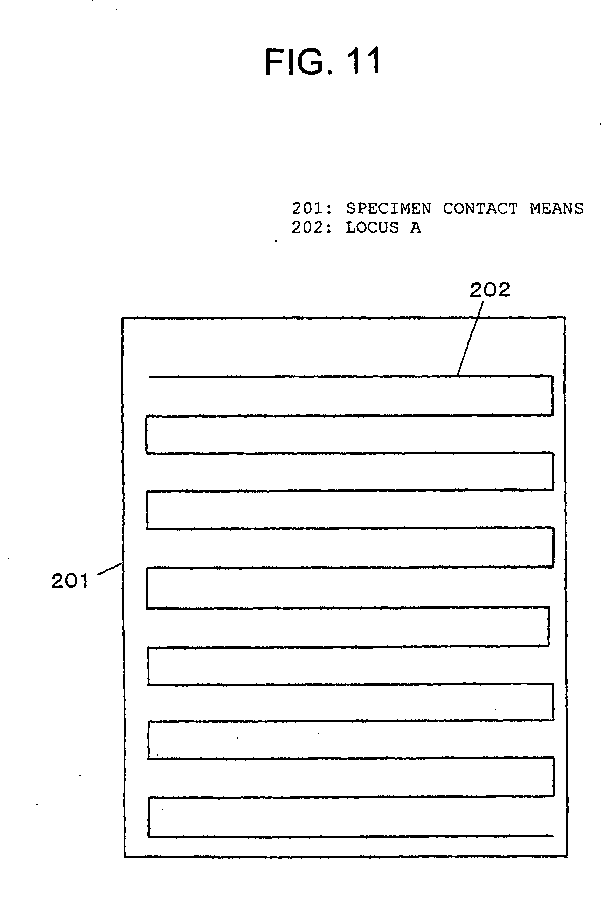

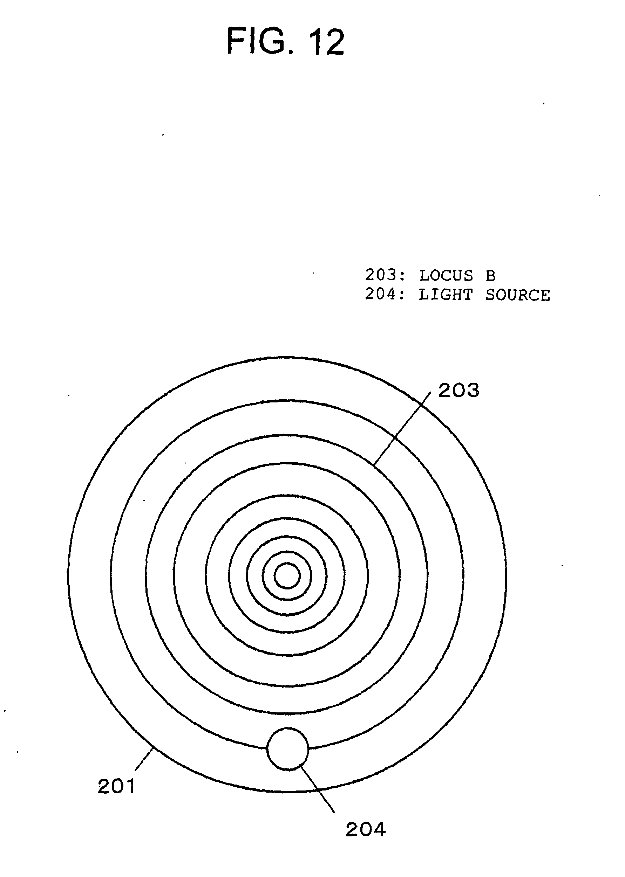

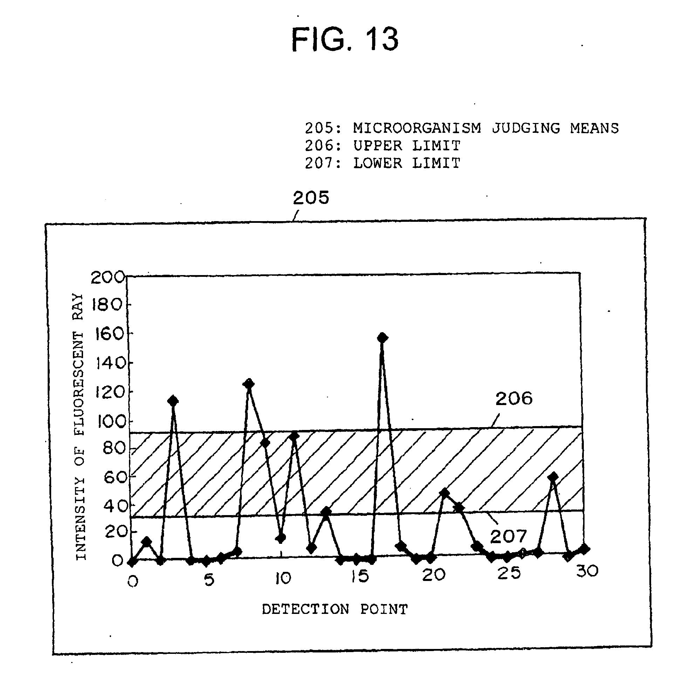

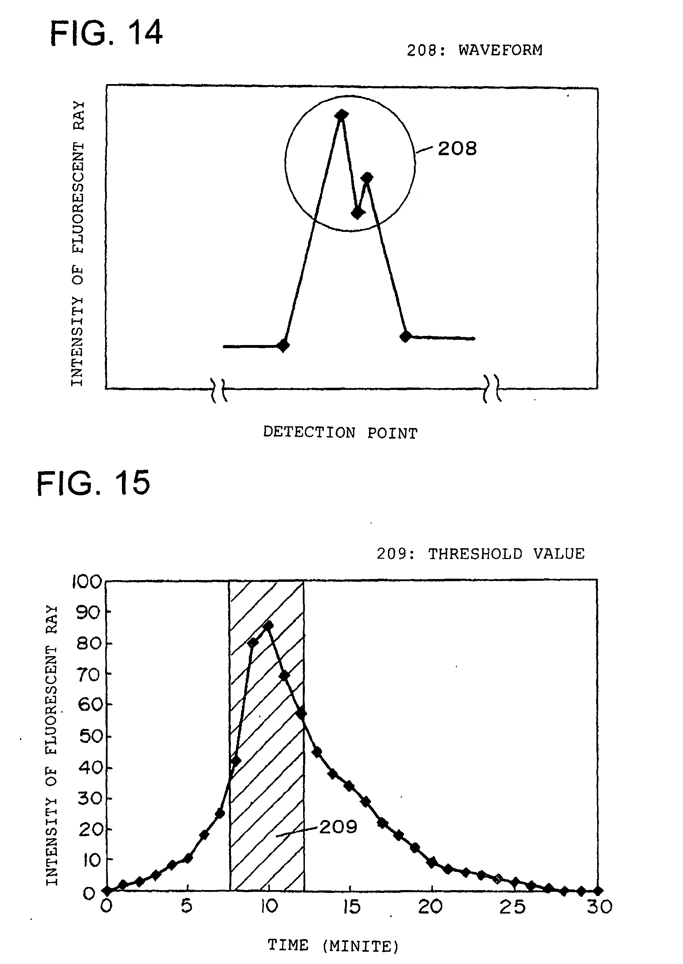

It is desired that a locus of exciting light on a specimen placed on a microorganism counting apparatus, as shown in Fig. 11, covers the entire specimen contact means 201. For this purpose, the exciting light is moved for detection as along a locus A202 to cover the width and the length of a specimen. The locus for detecting the entire specimen may be circular as shown in Fig. 12. Therefore, the exciting light can be moved as along a locus B203 to detect microorganisms. Shown in Fig.13 is a model of a microorganism judging means in which a light source 204 is moved along each of the loci on the specimen contact means, and using fluorescent rays produced by the exalting light emitted from the light source 204. Fluorescent rays emitted from a specimen forms a microorganism judging means 205 in correlation to sites emitting the fluorescent rays. Scattered rays produced from impurities or the like, fluorescent rays generated by the exciting light and the like coexist on the microorganism judging means 205, and rays are not same as fluorescent rays emitted from target microorganisms in some cases. Therefore, threshold values are provided. The threshold values include an upper limit value 206 and a lower limit value 207 for the intensity of fluorescent rays, and fluorescent rays within a range between the upper and lower limit values are recognized as derived from microorganisms and added all together. Thus, fluorescent rays derived from impurities other than microorganisms can be grasped to enable the precise counting of microorganisms. The threshold values are determined by the intensity of fluorescent rays emitted by microorganisms. When adjacent microorganisms exist, the waveform provided by fluorescent rays emitted from microorganisms is of a profile indicating the existence of the adjacent microorganisms as shown in Fig. 14. In this case, when the adjacent microorganisms have been detected in an agar culture medium diffusion process which is one of the prior art processes, colonies may be superposed one on another with the propagation of the microorganisms and the coalescence of colonies and determined as one colony at a time point of confirmation visually in some cases. Therefore, according to the present invention, in a case as shown in the waveform 208, the adjacent microorganisms are detected as one microorganism. Thus, it is possible to provide the correlation to the agar culture medium diffusion process.

The time of application of exciting light to the specimen contact means 201 is set in a range in which the intensity of fluorescent rays is not extinguished, as shown in Fig. 15. Namely, the time is in a range of threshold values 209.

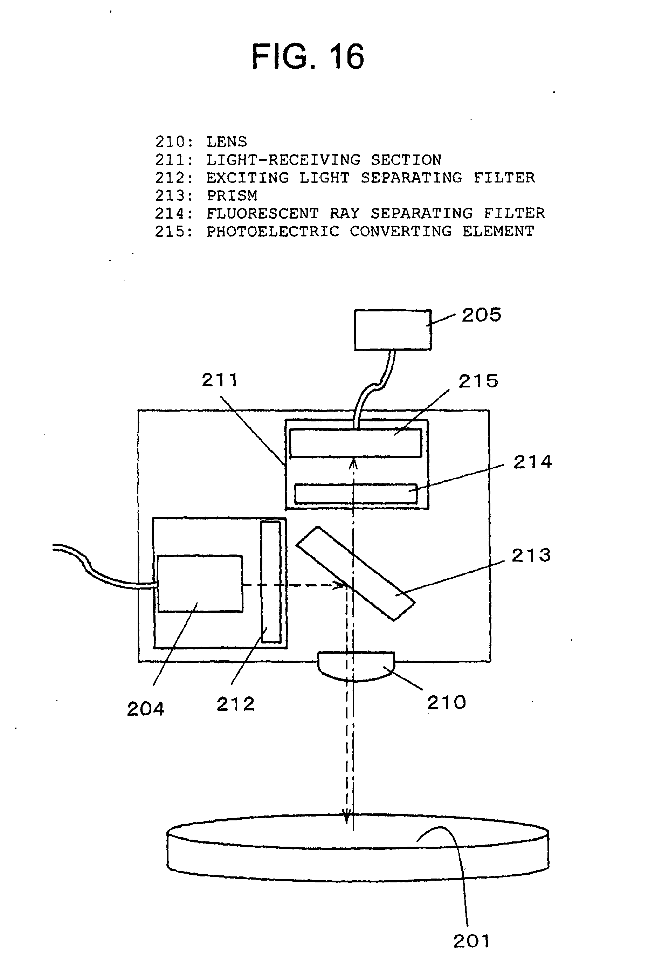

As shown in Fig. 16, the microorganism counting apparatus comprises a light source 204, a lens 210 as a light collector means, and a light-receiving section 211. Exciting light emitted from the light source 204 is separated by an exciting light separating filter 212 to extract a target wavelength. The separated exciting light is diverted through a prism 213 into a path. The diverted exciting light is passed through the lens 210 and collected onto the surface of a specimen contact means 201. Fluorescent rays emitted by microorganisms excited on such surface by the exciting light are transmitted again through the prism 213. In this case, the fluorescent rays are transmitted, as they are, through the prism 213 to reach the light-receiving section 211. The fluorescent rays reaching the light-receiving section 211 are passed through a fluorescent ray separating filter 214 to extract only a target fluorescent ray, and to reach a photoelectric converting element 215 included in the light-receiving section 211, where they are converted into signals and recognized. Although not shown, the specimen contact means 201 or the microorganism counting apparatus is provided with a means for moving the specimen contact means, so that all or some of fluorescent colors developed in the specimen contact means 201 can be received.

The fluorescent rays reaching the photoelectric converting element 215 are judged as derived from microorganisms or foreign matters in the microorganism judging means 205. The fluorescent rays judged as derived from microorganisms are added all together, and the number of microorganisms is counted.

The exciting light emitted from the light source 204 is collected by the lens 210, and the exciting light to be applied by the lens 210 is collected in a region of a very small given area. In this case, the term "very small given area" indicates a region on the order of 0.2 to 7.0 µm per side, as determined based on the size of bacteria or true fungi. On the basis of comparison with the agar culture medium diffusion process which is one of microorganism detecting means utilized currently in most oases, colonies formed by the group of microorganisms cultured and propagated by the agar culture medium diffusion process may be superposed one on another if they are closer in distance to one another and finally, the superposed colonies may be recognized as one colony as confirmed visually in some oases. In this case, the term "very small given area" indicates a region on the order of 100 to 500 µm per side, when it is based on the distance between the non-superposed colonies.

The time of application of the exciting light collected by the lens 210 depends on the time of extinction of the compound emitting fluorescent light and the intensity of the exalting light. Depending on the type of the compound, the compound may be decomposed even by ultraviolet rays existing in the natural world and hence, it is desirable that the exalting light is applied for a time in a range of about 2 second to about 300 seconds.

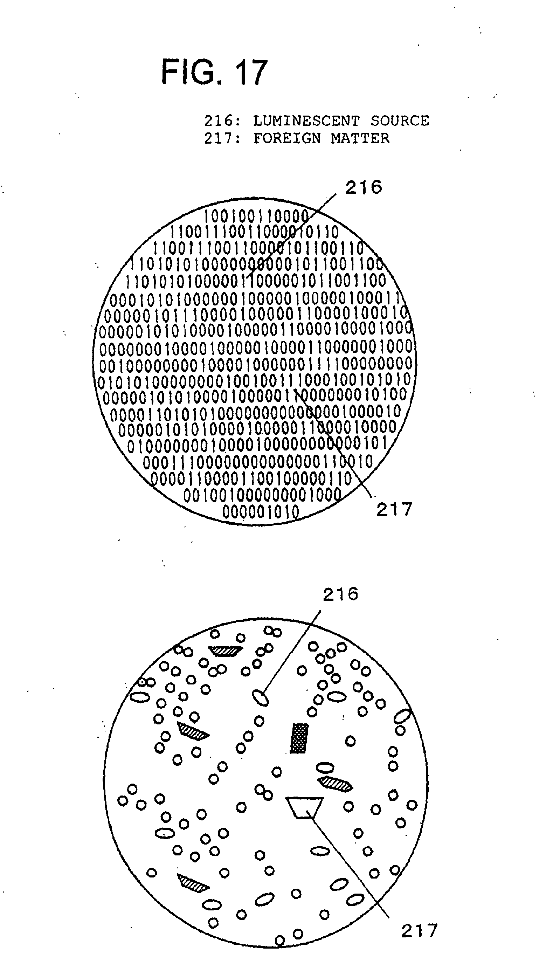

When the luminance after the coloration is recognized, a position of fluorescent coloration is represented by a binary system comprising "0" and "1" , as shown in Fig.17. In the binary system, adjacent colors developed are regarded as one luminescent source 216. If the size and the luminance of the luminescent source are relatively largely different from preset values, such luminescent source is recognized as a foreign matter 217 and regarded as out of the examination and is not counted as microorganism. As for a distance between adjacent colors developed, on the basis of comparison with the agar culture medium diffusion process which is one of microorganism detecting means utilized currently in most oases, colonies formed by the group of microorganisms cultured and propagated by the agar culture medium diffusion process may be superposed one on another if they are closer in distance to one another and finally, the superposed colonies may be recognized as one colony as confirmed visually in some oases. Thus, the term "very small given area" indicates a region on the order of 100 to 500 µm per side, when it is based on the distance between the non-superposed colonies.

Microorganisms deposited on a specimen emit fluorescent rays by virtue of the coloring compound.

The luminescence relies on the activity of an enzyme in the cells and is capable of dyeing living germs. Reagents for dyeing Coliform bacteria microorganisms are 4-methylumbelliferyl-β-D-galactoside and 4-methylumbelliferyl-β-D-gluouronide. These compounds react specifically with an enzyme produced by Coliform bacteria to become decomposed into 4-methylumbelliferron. The 4-methylumbelliferron is excited by ultraviolet rays to emit fluorescent light. Thus, the number or the presence or absence of Coliform bacteria can be detected by detecting the fluorescent light emitted by the excitation by the ultraviolet rays.

A means for detecting microorganisms after being colored or dyed is provided. The compounds have different maximum exciting wavelengths and emit fluorescent rays having different wavelengths, after being excited, thereby enabling the multiplex dyeing. In the detection of colors developed, if the light from the light source 204 has a wider wavelength, the exciting light can be regulated in wavelength and separated by the exciting light separating filter 212. The exciting light separating filter 212 can be exchanged depending on a target subject to be detected and hence, it is possible to accommodate various fluorescent reagents. At the same time, if the fluorescent colored light has a wider wavelength, it is possible to accommodate various fluorescent reagents by exchanging the fluorescent light separating filter 214 depending on a target subject to be detected in order to detect a target color developed.

Examples of the light source 204 are various diodes, a halogen lamp, a xenon lamp, a cold cathode tube, a laser, a black light, a mercury lamp and the like. Among these light sources, any of the diode, the cold cathode tube and the black light with the maximum exciting wavelength limited relatively can be mounted without use of the exciting light separating filter 212 and the fluorescent light separating filter 214 in some oases. When the halogen lamp, the mercury lamp or the like is used, the use of the exciting light separating filter 212 and the fluorescent light separating filter 214 may be required in some cases.

The prism 213 and the lens 210 have a nature that they transmit ultraviolet rays, as required. Examples of materials having the ultraviolet ray-transmittable nature for forming the prism 213 and the lens 210 are a quartz glass and the like. Thus, it is possible to accommodate fluorescent reagents excited by ultraviolet rays. The specimen contact means 201 may be placed on a member which is rotatable. The exciting light collected by the lens 210 is moved from an outer peripheral portion to a center portion of the specimen contact means 201, or from a center portion to an outer peripheral portion of the specimen contact means 201 through a distance corresponding to the radius of specimen contact means 201. In this case, it is possible to prevent the occurrences of the slippage, the persistence of vision and the afterglow of the fluorescent light emitted by the excited compound when the exciting light collected by the lens 210 exists at the outer peripheral portion and when the exciting light collected by the lens 210 exists at the center portion, by changing the rotating speed of the specimen contact means 201 when the position of the exciting light collected by the lens 210 is at the outer peripheral portion and when the position of the exciting light collected by the lens 210 is at the center portion.

A means is provided for recognizing the position in which the exciting light has been collected. Thus, the position of the exciting light collected by the lens 210 is recognized, so that the collection of the light is prevented from being deviated from the locus, and when the collection of the light has been deviated, it is returned again to the locus.

The coloring or staining reagents having the cell permeability have been exemplified, but any of coloring or staining reagents may be used which have a cell membrane depositability, a call wall depositability, a sugar chain bondability, a functional group bondability, an amino acid adherability, an ion sensitivity, an oxygen reactivity, and even in this case, cells can be likewise detected.

The coloring or staining reagents excited by the exciting light to emit the fluorescent light have been exemplified above.

The very small given area, to which the exciting light is applied, is not limited to the polygonal shapes including a square shape, and may be circular, elliptic or the like, if a specimen can be irradiated.

The compounds contained in the specimen contact means are capable of carrying out the multiplex dyeing, but they are not necessarily required to be mixed together. For example, a single specimen contact means maybe divided into portions corresponding to the number of dyeing compounds, so that cells can be detected in such portions, respectively.

A diffraction grating may be utilized as a means for separating the exciting light or the fluorescent light.

The persistence of vision and the afterglow of the fluorescent light are prevented by adjusting the rotating speed of the specimen contact means 201, but may be prevented by adjusting the moving speed of the lens 210 for applying the exciting light.

The means for recognizing the light collection position is not necessarily required to directly recognize the exciting light collection position, and may be a means capable of grasping the locus on the specimen contact means 201.

Example 11 of the present invention will be described below.

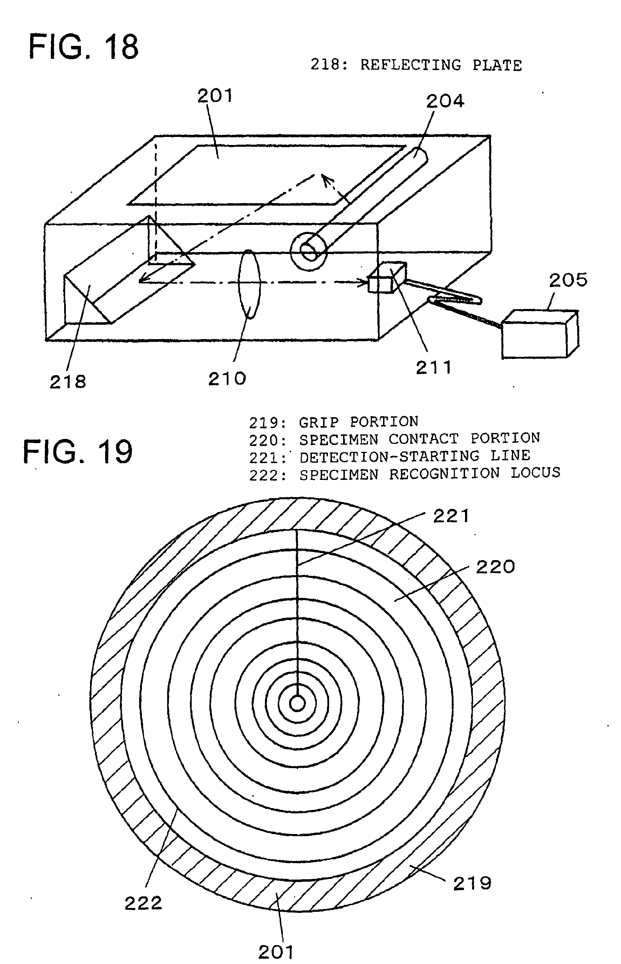

As shown in Fig.18, a microorganism counting apparatus comprises a specimen contact means 201, a light source 204, a reflecting plate 218, a lens 210, and a light-receiving section 211. Fluorescent rays excited and produced from dyed microorganisms existing in a specimen by the exiting light emitted from the light source 204 are turned in direction by the reflecting plate 218, collected by the lens 210 and detected by the light-receiving section 211. The fluorescent rays reaching the light-receiving section 211 are judged as microorganisms or foreign matters in a microorganism judging means 205. The fluorescent rays judged as the microorganisms are added all together, whereby the number of the microorganisms is counted. The light source 204 scans the specimen contact means 201. Further, the reflecting plate 218 is placed in correspondence to the locus of the light source 204.

The exciting light emitted from the light source 204 is collected by the lens 210, and the exciting light to be applied is collected in a region of a very small given area. In this case, the term "very small given area" indicates a region on the order of 0.2 to 7.0 µm per side, as determined based on the size of Coliform bacteria. On the basis of comparison with the agar culture medium diffusion process which is one of microorganism detecting means utilized currently in most oases, colonies formed by the group of microorganisms cultured and propagated by the agar culture medium diffusion process may be superposed one on another if they are closer in distance to one another and finally, the superposed colonies may be recognized as one colony as confirmed visually in some cases. In this case, the term "very small given area" indicates a region on the order of 100 to 500 µm per side, when it is based on the distance between the non-superposed colonies.

The time of application of the exciting light collected by the lens 210 depends on the time of extinction of the compound emitting fluorescent light and the intensity of the exciting light. Depending on the type of the compound, the compound may be decomposed even by ultraviolet rays existing in the natural world and hence, it is desirable that the exciting light is applied for a time in a range of about 2 second to about 300 seconds.

Examples of the light source 204 are various diodes, a halogen lamp, a mercury lamp, a xenon lamp, a cold cathode tube, a laser, a black light and the like.

The reflecting plate 218 and the lens 210 have a nature that they transmit ultraviolet rays, as required. Examples of materials having the ultraviolet ray-transmittable nature for forming the reflecting plate 218 and the lens 210 are a quartz glass and the like. Thus, it is possible to accommodate fluorescent reagents excited by ultraviolet rays.

In order to prevent any influence to the fluorescent light due to a reduction in intensity of the exciting light emitted from the light source 204 to the specimen contact means 201 or due to external light, a dark-field view plate or the like for placing the specimen contact means 201 in a dark-field environment may be placed.

The light source 204 is not necessarily movable. It is possible to detect the deposited microorganisms by moving the specimen contact means 201.

The light source 204 is not necessarily built in the microorganism counting apparatus. A light source section including a light source 204 maybe mounted outside the apparatus, so that exciting light can be introduced from the light source section into the apparatus utilizing an optical fiber or the like.

A means for removing lights of wavelengths other than a target wavelength may be provided depending on the type of the light source. Examples of such means are an exciting light separating filter, a diffraction grating and the like.

In addition, a means for removing lights of wavelengths other than a target wavelength may be provided depending on the type of fluorescent light emitted. Examples of such means are a fluorescent light separating filter, a diffraction grating and the like.

Further, a prism may be utilized in place of the reflecting plate 218.

Example 12 of the present invention will be described below.

As shown in Fig. 19, a specimen contact means 201 comprises a grip portion 219 for enhancing the handleability for an operator, a specimen contact portion 220 for deposition of microorganisms from s subject of detection, a detection-starting line 221 for recognizing a detection-starting point, and a specimen recognition locus 222 for recognizing a detecting locus.

An operator takes a grip portion 219 by his hand and pushes it against a target subject of examination for several seconds. At this time, the pushing is carried out at a strength and for a time as much as the specimen contact portion 220 is not crashed. Microorganisms deposited from the subject of examination are dyed by the addition of a compound for staining or dyeing microorganisms depending on the state or nature of the microorganisms or by the permeation of a compound previously contained in the specimen contact portion 220. The detection of the specimen contact means 201 containing the dyed microorganisms is started. The specimen recognition locus 222 previously provided in the specimen contact means 201 to recognize the position of the exciting light collected on the specimen contact means 201 is detected to recognize the position of the exciting light. The exciting light collected by the lens 210 shown in Fig.16 is moved around an outer periphery from a start point on the detection-starting line 221. After movement of the exciting light around the outer periphery, when the exciting light again reaches the detection-starting line 221, the exiting light collected by the lens 210 shown in Fig.16. is diverted into a next inner portion of the specimen recognition locus 222, and thus, the number of colors Included in such inner locus portion is recognized. Such a movement is repeated, namely, the exciting light is moved toward the center, whereby the number of microorganisms included in an area where the detection is required is detected.

It is desirable that a substance used in the specimen contact portion 220 is one which does not influence fluorescent light. It is also desirable to use a substance having a self-adherent property in order to efficiently bring microorganisms into contact with the specimen contact portion 220 from a specimen. Examples of such substances are various polymers such as gum arabic, acrylic resins and the like, substances containing moisture moderately and having a self-adherent property, such as agar, and the like.

Examples of the detection-starting line 221 are a marking out directly in the specimen contact means, a marker capable of recognizing the exciting light or a light-collecting position and having a reflectivity. This also applies to the specimen recognition locus 222, but it is desirable to use a marker which does not influence fluorescent light.

The grip portion is not necessarily provided on the outer periphery.

What recognizes the detection-starting point is not limited to the line.

The detection locus may be spiral or oblique.

Further, the start point for the movement of the exciting light is not limited to the outer periphery.

Example 13 of the present invention will be described below, (not included in the invention).

In a microorganism counting process, first, 4' , 6-diamidino-2-phenylindole dihydrochloride which is a first compound and propidium iodide which is a second compound are brought in the form of respective solutions or in the form of a reagent mixture into contact with a specimen having cells or microorganisms deposited thereon. The 4',6-diamidino-2-phenylindole dihydrochloride is passed through cell membranes on the specimen and bonded to nucleic acid. The propidium iodide cannot be passed through living cell membranes because of its large molecular weight, and is bonded to nucleic acid in dead cells.

The 4',6-diamidino-2-phenylindole dihydrochloride bonded to nucleic acid in living and dead cells absorbs light having an exciting wavelength of 359 nm to emit fluorescent light having a wavelength of 461 nm, thereby coloring or dyeing the living and dead cells. The propidium iodide bonded to nucleic acid in dead cells absorbs light having an exciting wavelength of 535 nm to emit fluorescent light having a wavelength of 617 nm, thereby coloring or dyeing the dead cells. The colored cells are counted visually or by a counter, whereby the number of the living and dead cells and the number of the dead cells are confirmed.

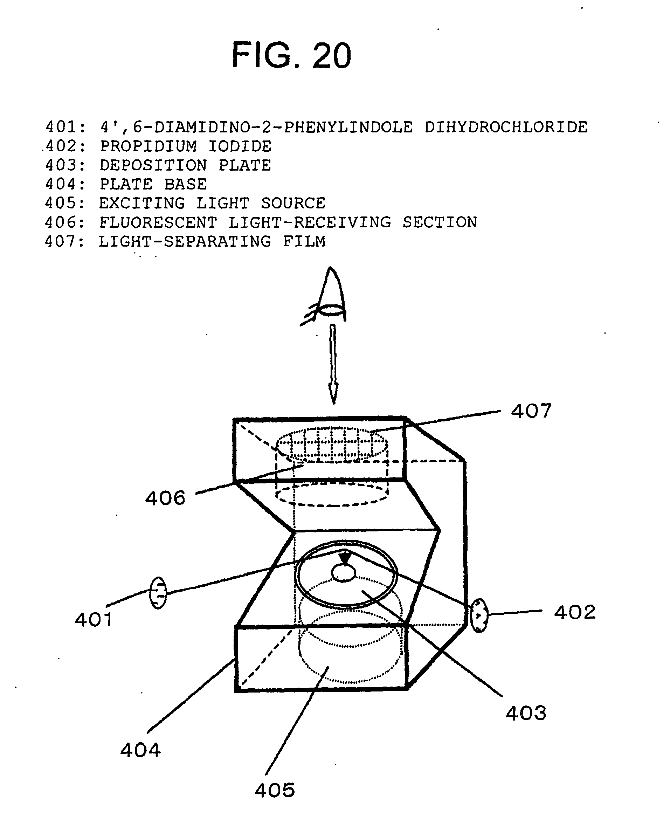

The microorganism counting process will be described with reference to Fig.20. In the microorganism counting process, 4',6-diamidino-2-phenylindole dihydrochloride 401 and propidium iodide 402 are used as detecting reagents, and an apparatus is used, which is comprised of a deposition plate 403 made of glass and having cells or microorganisms in a specimen deposited thereon, a plate base 404 on which the deposition plate 403 is placed, an exciting light source 405 mounted at a lower portion of the plate base 404 for applying exciting light to the deposition pate 403, a fluorescent light-receiving section 406 including a magnifying mirror for confirming fluorescent colors developed by cells or microorganisms in the specimen, and a light-separating film 407 mounted at an upper portion of the fluorescent light-receiving section 406 for limiting the wavelength of fluorescent light.

In the counting process, a detecting reagent mixture prepared by mixing 4',6-diamidino-2-phenylindole dihydrochloride 401 and propidium iodide 402 is used. A solution containing cells or microorganisms collected from a specimen is deposited or dropped onto the surface of the deposition plate 403; the deposition plate 403 is placed onto the plate base 404, and the detecting reagent mixture is dropped onto the surface of the deposition plate 403. The exciting light source 405 mounted at the lower portion of the deposition plate 403 is turned on, whereby the cells or microorganisms on the deposition plate 403 are stained or colored in a fluorescent manner, and fluorescent colors developed are magnified by the fluorescent light-receiving section 406. Further, by the light-separating film 407 transmits light having particular wavelengths, the cells or microorganisms emitting fluorescent rays are lighted individually. The cells or microorganisms lighted at the particular wavelengths are counted, whereby the number of living and dead cells and the number of dead cells are counted and thus, the number of existing living cells is calculated. In this manner, the number of the living cells and the number of the dead cells in the specimen can be confirmed.

The fluorescent light-receiving section 406 is provided with a lens of 200 to 1,000 magnifications.

Example 14 of the present invention will be described below (not included in the invention).

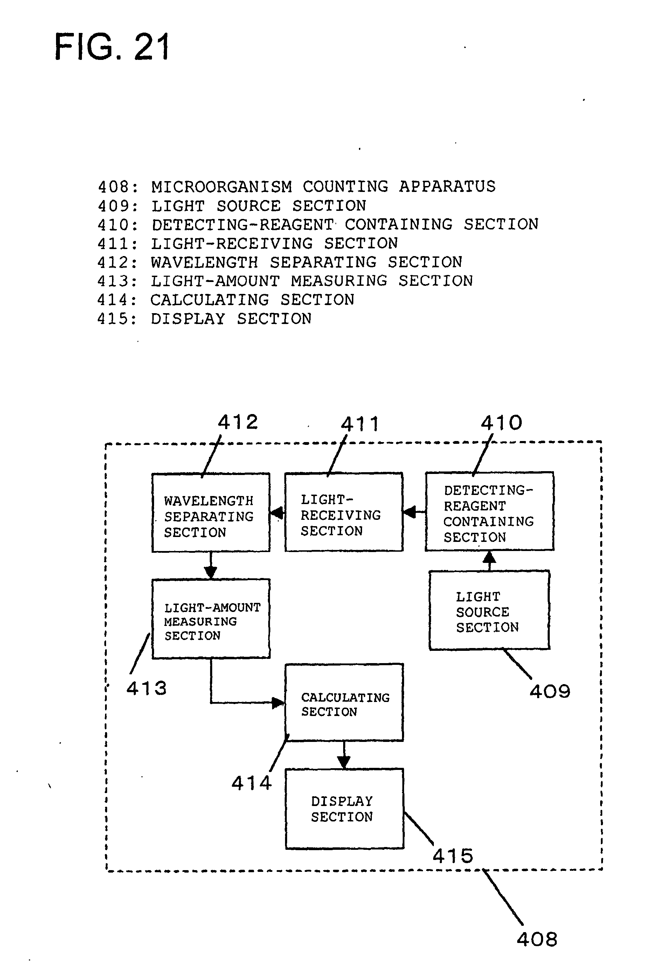

A block diagram of an apparatus for carrying out the counting automatically is shown in Fig. 21. As shown in Fig. 21, the microorganism counting apparatus 408 is comprised of a light source section 409 including a lamp for emitting exciting light, a detecting-reagent containing section 410 made of a glass, in which a solution containing 4',6-diamidino-2-phenylindole dihydrochloride 401 as a detecting reagent for staining or coloring living and dead cells of microorganisms collected from a specimen and propidium iodide 402 as a detecting reagent for staining or coloring dead cells of microorganisms is incorporated, a light-receiving section 411 for receiving colored light, a wavelength separating section 412 for separating light from the light-receiving section 411, a light-amount measuring section 413 for measuring an amount of light, a calculating section 414 for calculating the numbers of living calls and the dead cells, and a display section 415 for displaying the calculated numbers of the cells.

An example of use of the microorganism counting apparatus 408 will be described below.

In the use of the microorganism counting apparatus 408, 0.5 grams of vegetables are dipped into 9. 5 ml of a physiological saline to prepare a suspension. Then, 1 ml of the suspension, 4',6-diamidino-2-phenylindole dihydrochloride 401 and propidium iodide 402 are incorporated into the detecting-reagent containing section 410. After the incorporation, the detecting reagents and the suspension contained in the detecting-reagent containing section 410 are agitated automatically, whereby the detecting reagents are bonded to microorganism cells in the suspension. The lamp included in the light source section 409 for emitting exciting light is turned on to apply exciting light automatically to a specimen, whereby nucleic acid in living and dead cells bonded with the detecting reagents and nucleic acid in dead cells bonded with the detecting reagents emit rays, which are applied to the light-receiving section 411. The rays applied to the light-receiving section 411 are separated in the wavelength separating section 412 into rays having a fluorescent wavelength of 450 to 480 nm indicative of living and dead cells and rays having a fluorescent wavelength of 500 to 620 nm indicative of dead cells, and amounts of rays are converted into electric signals in the light-amount measuring section 413. The resulting electric signals are fed to the calculating section 414, where the numbers of cells are calculated from a calibration curve for number of cells and electric signals, and the number of the dead cells is subtracted from the number of the living and dead cells, thus calculating the number of the living cells. In this manner, the number of the living calls and the number of the dead cells in 1 ml of the suspension incorporated in the detecting-reagent containing section 410 can be displayed automatically on the display section 415.

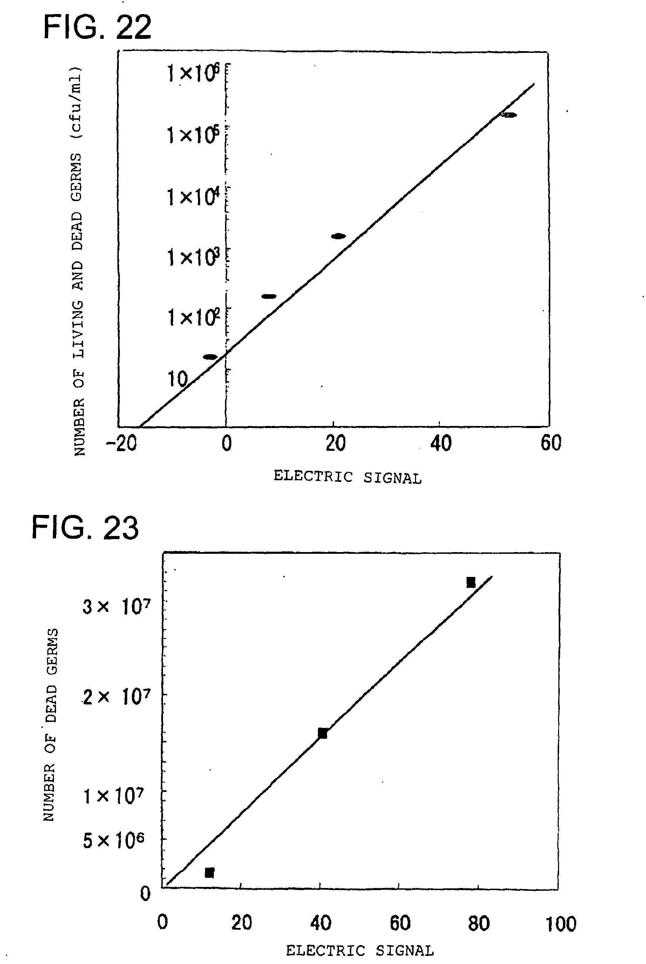

A graph of a calibration curve for living and dead cells and electric signal output is shown in Fig.22, and a graph of a calibration curve for dead cells and electric signal output is shown in Fig.23.

The amount of 4',6-diamidino-2-phenylindole dihydrochloride 401 dropped or added to the suspension is in a range of 10 n mole/ml (inclusive) to 1 µ mole/ml (inclusive) in terms of a final concentration with respect to the amount of a specimen, and an optimal concentration is 100 n mole/ml. The amount of propidium iodide 402 dropped or added to the suspension is in a range of 10 n mole/ml (inclusive) to 1µ mole/ml (inclusive) in terms of a final concentration with respect to the amount of a specimen, and an optimal concentration is 100 n mole/ml.

The exciting light source 405 used is one adapted to emit light having an exciting wavelength in a range of 400 to 700 nm.

According to the present invention, the number of cells or microorganisms in a specimen can be detected visually or automatically, promptly and precisely by using the fluorescent coloration.

The deposition plate 403 made of a glass is used in Example 13, but a material such as an acrylic resin and a polymer film capable of transmitting exciting light may be used.

The amounts of rays has been converted into electric signals to count the number of cells in Examples 13 and 14, but a process which comprises picking up pictures to count the number of cells in terms of the amount of lighted pictures may be used.

The detecting-reagent containing section 410 made of a glass is used in Example 14, but a material capable of transmitting exciting light and fluorescent light may be used.

Example 15 of the present invention will be described below.

In a microorganism counting process, first, a reagent mixture made by mixing 4',6-diamidino-2-phenylindole dihydrochloride as a first compound, propidium iodide as a second compound, 6-carboxyfluorescein diacetate as a third compound and 4-methylumbelliferyl-β-D-galaotoside as a fourth compound is brought into a specimen having cells or microorganisms deposited thereto. The 4',6-diamidino-2-phenylindole dihydrochloride bonded to nucleic acid in living and dead cells absorbs exciting light having an exciting wavelength of 359 nm to emit fluorescent light having a wavelength of 461 nm to color living and dead cells. The propidium iodide bonded to nucleic acid in dead cells absorbs exciting light having an exciting wavelength of 535 nm to emit fluorescent light having a wavelength of 617 nm to color only dead cells. The 6-carboxyfluorescein diacetate reacting with esterase in living cells is decomposed by the esterase to produce fluorescein, which absorbs exciting light having an exciting wavelength of 474 nm to emit fluorescent light having a wavelength of 541 nm to color only living cells. The 4-methylumbelliferyl-β-D-galactoside reacts with β-galactosidase produced as a metabolite from Coliform bacteria to produce 4-methylumbelliferone, which absorbs exciting light having an exciting wavelength of 394 nm to emit fluorescent light having a wavelength of 489 nm to color only Coliform bacteria. The number of living and dead cells and the number of dead cells are counted from the number of the colored cells and thus, the numbers of the living cells and the dead cells are calculated. Further, the presence or absence of living cells, and living Coliform bacteria as particular microorganisms can be confirmed.

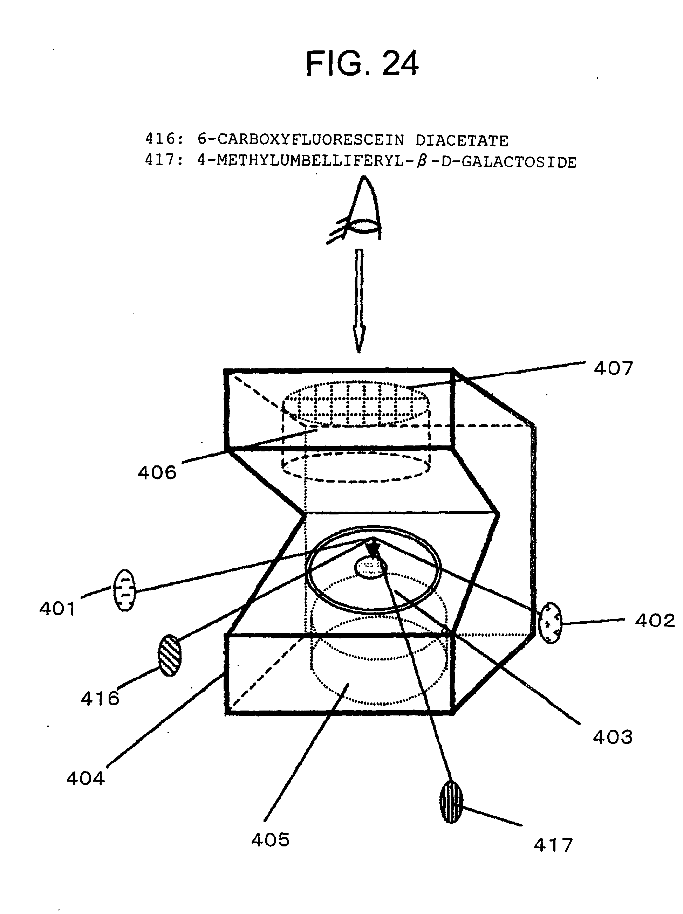

The microorganism counting process of the present invention shown in Fig.24 will be described below. In the microorganism counting process, 4' ,6-diamidino-2-phenylindole dihydrochloride 401, propidium iodide 402, 6-carboxyfluorescein diacetate 416 and 4-methylumbelliferyl-β-D-galactoside 417 are used as detecting reagents, and an apparatus is used, which is comprised of a deposition plate 403 made of glass and having cells or microorganisms deposited thereon, a plate base 404 on which the deposition plate 403 is placed, an exciting light source 405 mounted at a lower portion of the plate base 404 for applying exciting light to the deposition pate 403, a fluorescent light-receiving section 406 mounted above the deposition plate 404 and including a magnifying mirror for catching colors developed by cells or microorganisms in the specimen, and a light-separating film 407 mounted at an upper portion of the fluorescent light-receiving section 406 for limiting the wavelength of fluorescent light.

In the counting process, a detecting reagent mixture prepared by mixing 4',6-diamidino-2-phenylindole dihydrochloride 401, propidium iodide 402, 6-carboxyfluoresesin diacetate 416 and 4-methylumbelliferyl-β-D-galactoside 417 is used. The deposition plate 403 with a solution deposited thereon and containing cells or microorganisms collected from a specimen is placed onto the plate base 404, and the detecting reagent mixture is dropped onto the surface of the deposition plate 403. The exciting light source 405 mounted at the lower portion of the deposition plate 403 is turned on, whereby the cells or microorganisms on the deposition plate 403 are stained or colored in a fluorescent manner, and the fluorescent rays are magnified and transmitted by the fluorescent light-receiving section 406. Further, by the light-separating film 407 transmits light having particular wavelengths, the cells or microorganisms emitting fluorescent rays are lighted individually. Thus, the living and dead cells and the dead cells can be confirmed visually, and further, the presence or absence of highly active living cells and Coliform bacteria can be confirmed visually.

When the living and dead cells are counted, the light-separating film 407 capable of transmitting fluorescent light having a wavelength in a range of 450 to 480 nm is used. When the dead cells are counted, the light-separating film 407 capable of transmitting fluorescent light having a wavelength in a range of 500 to 620 nm is used. When the living cells are counted, the light-separating film 407 capable of transmitting fluorescent light having a wavelength in a range of 450 to 560 nm is used. When the Coliform bacteria are counted, the light-separating film 407 capable of transmitting fluorescent light having a wavelength in a range of 400 to 550 nm is used.

Example 16 of the present invention will be described below.

An apparatus for automatically counting microorganisms will be described with reference to Fig. 21, because it has an arrangement similar to that shown in Fig. 21. As shown in Fig. 21, the microorganism counting apparatus 408 is comprised of a light source section 409 including a lamp for emitting exciting light, a detecting-reagent containing section 410 made of a glass, in which a solution containing 4',6-diamidino-2-phenylindole dihydrochloride 401 as a detecting reagent for staining or coloring living and dead cells of microorganisms collected from a specimen and propidium iodide 402 as a detecting reagent for staining or coloring dead cells of microorganisms, 6-carboxyfluorescein diacetate 416 as a detecting reagent for staining or coloring living cells of microorganisms and 4-methylumbelliferyl-β-D-galactoside 417 as a detecting reagent for staining or coloring Coliform bacteria is incorporated, a light-receiving section 411 for receiving colored light, a wavelength separating section 412 f or separating light from the light-receiving section 411, a light-amount measuring section 413 for measuring an amount of light, a calculating section 414 for calculating the numbers of living cells and the dead cells or the number of Coliform bacteria, and a display section 415 for displaying the calculated numbers of the cells.

An example of use of the microorganism counting apparatus 408 will be described below.

In the use of the microorganism counting apparatus 408, 0.5 grams of vegetables are dipped into 9.5 ml of a physiological saline to prepare a suspension. Then, 1 ml of the suspension, 4',6-diamidino-2-phenylindole dihydrochloride 401, propidium iodide 402, 6-carboxyfluorescein diacetate 416 and 4-methylumbelliferyl-β-D-galactoside 417 are incorporated into the detecting-reagent containing section 410. After the incorporation, the detecting reagents and the suspension contained in the detecting-reagent containing section 410 are agitated automatically, whereby the detecting reagents are bonded to microorganism cells in the suspension. The lamp included in the light source section 409 for emitting exciting light is turned on to apply exciting light automatically to a specimen, whereby nucleic acid in living and dead cells bonded with the detecting reagents and nucleic acid in dead cells bonded with the detecting reagents are colored, and fluorescein and 4-methylubbelliferon are colored by the action of esterase in living cells and β-galactosidase produced as a metabolite from Coliform bacteria, thereby emitting rays to the light-receiving section 411. The rays applied to the light-receiving section 411 are separated in the wavelength separating section 412 into rays having a fluorescent wavelength of 461 nm indicative of living and dead cells, rays having a fluorescent wavelength of 617 nm indicative of dead cells, rays having a fluorescent wavelength of 541 nm indicative of living cells and rays having a fluorescent wavelength of 489 nm indicative of Coliform bacteria, and amounts of rays are converted into electric signals in the light-amount measuring section 413. The resulting electric signals are fed to the calculating section 414, where the numbers of cells are calculated from a calibration curve for number of cells and electric signals, and the number of the living cells is calculated from a difference between the number of the living and dead cells and the number of the dead cells. In this manner, the number of the living and dead cells, the number of the dead cells, the number of highly active living cells and the number of Coliform bacteria in 1 ml of the suspension incorporated in the detecting-reagent containing section 410 counting apparatus including a light source and alight-receiving means, so that cells are counted from a difference between wavelengths of rays emitted from a specimen and amounts of fluorescent colors developed. Thus, an artificial error can be minimized, and cells can be counted easily even by those not skilled in the art.

As can be seen from the above-described Examples it is possible to provide a microorganism counting process using the process of the invention comprising the steps of bringing one or more of a first compound for staining living and dead cells, a second compound for staining dead cells at a wavelength different from can be displayed automatically on the display section 415.

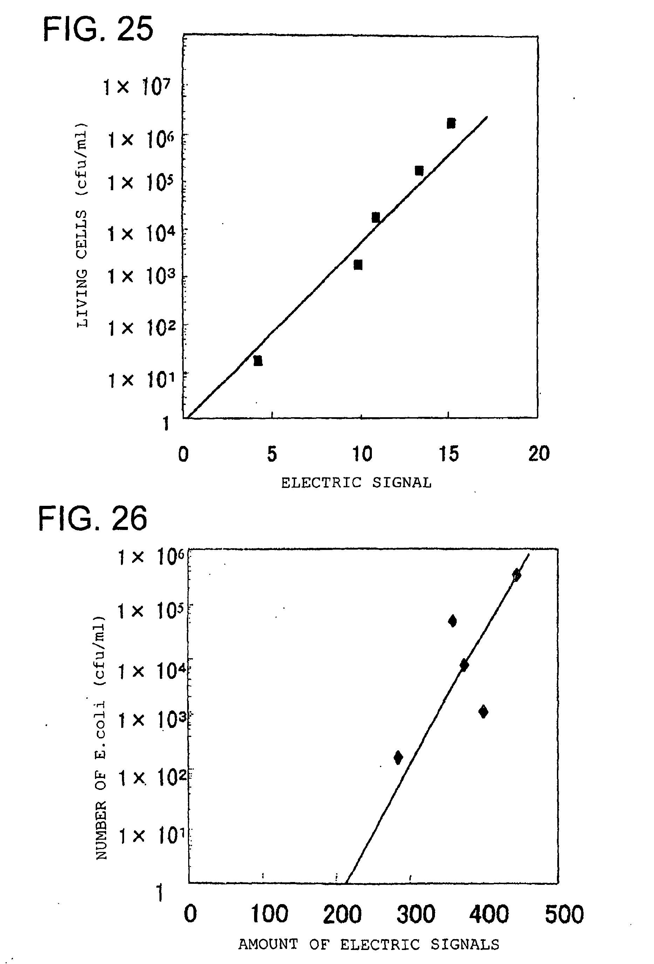

A graph of a calibration curve for highly active living cells and electric signal output is shown in Fig.25, and a graph of a calibration curve for Coliform bacteria and electric signal output is shown in Fig.26.

According to the present invention, the number of living cells and the number of dead cells can be counted by counting the number of living and dead cells and the number of dead cells by wavelengths of fluorescent colored rays, and can be detected promptly, simply and visually and further, the presence or absence of microorganisms such as Coliform bacteria and the number of highly active living cells can be confirmed.

### Industrial Applicability

As can be seen from the above-described Examples, according to the present invention, it is possible to provide a microorganism counting process comprising the steps of bringing one or more of a first compound for staining living and dead cells, a second compound for staining dead cells at a wavelength different from the above-described staining, and a third compound for staining living cells at a wavelength different from the above-described staining; and at least one or more fourth compounds for staining Coliform bacteria at a wavelength different from the above-described staining by the reaction with a substance derived from Coliform bacteria, into contact with a specimen, and detecting both or either one of living cells and dead cells, and Coliform bacteria simultaneously from a difference between wavelengths and amounts of colors developed. Thus, the monitoring of environments such as the pollution of foods, water or the like can be carried out at a real time, and the process can be utilized not only in a medical field but also in a food sanitation field. In addition, the presence of Coliform bacteria such as E. coli can be grasped promptly, thereby quickly preventing the influence to a market and a distribution due to the pollution of microorganisms.

In addition, it is possible to provide a microorganism counting process wherein the staining or coloration is fluorescent, thereby providing an effect that it is possible to prevent the mis-recognition from being provided by a measuring operator when light is received, and to count the number of cells precisely with a good accuracy.

Further, it is possible to provide a microorganism counting process wherein the substance derived from Coliform bacteria is an enzyme protein, thereby providing an effect that the presence or absence of special microorganisms can be confirmed, and microorganisms can be monitored. In addition, a pretreatment is not required and further, the examining time can be shortened.

Further, it is possible to provide a microorganism counting process comprising the step of bringing a compound for staining Coliform bacteria by the reaction with a substance derived from Coliform bacteria into contact with a specimen, and detecting Coliform bacteria from a wavelength and an amount of color developed. This provides an effect that only the Coliform bacteria can be counted quickly and hence, it is possible to grasp the polluted situation of foods and to previously prevent the incorporation of the Coliform bacteria to a human body.

## Claims

1. A microorganism counting process comprising the steps of bringing 4-methylumbelliferyl-β-D-galactoside and/or 4-methylumbelliferyl-β-D-glucuronide for coloring Coliform bacteria into contact with a specimen, and detecting each one of the Coliform bacteria by detecting the fluorescent light emitted by the excitation by ultraviolet rays, wherein said detection is performed by applying the exciting light which is emitted from a light source section and collected in a region of a very small given area of 0.2 to 7.0 µm per side as determined based on the size of Coliform bacteria and counting the number of Coliform bacteria based on the wavelength of the fluorescent coloured rays derived from 4-methylumbelliferron which is a decomposition product of 4-methylumbelliferyl-β-D-galactoside and/or 4-methylumbelliferyl-β-D-glucuronide in each Coliform bacteria in a light-amount measuring section converting the rays into electric signals.

## Patentansprüche

1. Verfahren zum Zählen von Mikroorganismen, umfassend die Schritte des in Kontakt Bringens von 4-Methylumbelliferyl-β-D-Galaktosid und/oder 4-Methylumbelliferyl-β-D-Glukuronid zum Anfärben von Coliform-Bakterien mit einer Probe und des
Detektierens vom jedem Coliform-Bakterium durch Detektieren des Fluoreszenzlichts, das durch die Anregung mit Ultraviolettstrahlen emittiert wird,
wobei die Detektion
durch Zuführen des Anregungslichts durchgeführt wird, das aus einem Lichtquellenabschnitt emittiert und in einer Region eines sehr kleinen bestimmten Bereichs von 0,2 bis 7,0 µm pro Seite wie festgelegt gesammelt wird, basierend auf der Größe der Coliform-Bakterien, und
durch Zählen der Anzahl der Coliform-Bakterien, basierend auf der Wellenlänge der fluoreszierenden gefärbten Strahlen, die von 4-Methylumbelliferron stammen, das ein Zersetzungsprodukt des 4-Methylumbelliferyl-β-D-Galaktosids und/oder des 4-Methylumbelliferyl-β-D-Glukuronids in jedem Coliform-Bakterium ist, in einem Licht-Mengenmessabschnitt, in welchem die Strahlen in elektrische Signale umgewandelt werden.

## Revendications

1. Procédé de comptage de microorganismes comprenant les étapes qui consistent à mettre en contact avec un spécimen du 4-méthylumbelliferyl-β-D-galactoside et/ou du 4-méthyl-umbelliferyl-β-D-glucuronide, pour colorer des bactéries coliformes, et à détecter chacune des bactéries coliformes en détectant la lumière fluorescente émise par l'excitation de rayons ultraviolets, la détection étant effectuée en appliquant la lumière d'excitation émise par une section de source lumineuse et collectée dans une zone d'une très petite superficie donnée allant de 0,2 à 7,0 µm par côté, déterminée sur la base de la taille des bactéries coliformes et en comptant le nombre de bactéries coliformes sur la base de la longueur d'onde des rayons colorés fluorescents dérivés du 4-méthyl-umbelliferone, qui est un produit de décomposition du 4-méthyl-umbelliferyl-β-D-galactoside et/ou du 4-méthyl-umbelliferyl-β-D-glucuronide dans chaque bactérie coliforme, dans une section de mesure de quantité de lumière convertissant les rayons en signaux électriques.
